# EUROPÄISCHE PATENTSCHRIFT

(11) **EP 3 637 362 B1**
(45) Veröffentlichungstag und Bekanntmachung des Hinweises auf die Patenterteilung: **11.03.2026**
(21) Anmeldenummer: 18199877.4
(22) Anmeldetag: 11.10.2018
(51) Int. Cl.: G06T 5/00

(54) **VERFAHREN ZUR ANPASSUNG EINES BILDEINDRUCKS**
METHOD FOR ADJUSTING AN IMAGE IMPRESSION
PROCÉDÉ D'AJUSTEMENT D'UNE IMPRESSION D'IMAGE

(43) Veröffentlichungstag der Anmeldung: 15.04.2020
(73) Patentinhaber: Siemens Healthineers AG, 91301 Forchheim (DE)
(72) Erfinder: Birkhold, Annette, 90429 Nürnberg (DE); Kaethner, Christian, 91301 Forchheim (DE); Kowarschik, Markus, 90408 Nürnberg (DE)
(74) Vertreter: Siemens Healthineers Patent Attorneys

(56) Entgegenhaltungen:
- YUBIN DENG ET AL: "Aesthetic-Driven Image Enhancement by Adversarial Learning", ARXIV.ORG, CORNELL UNIVERSITY LIBRARY, 201 OLIN LIBRARY CORNELL UNIVERSITY ITHACA, NY 14853, 17 July 2017 (2017-07-17), XP080777346
- MANSOOR AWAIS ET AL: "Adversarial approach to diagnostic quality volumetric image enhancement", 2018 IEEE 15TH INTERNATIONAL SYMPOSIUM ON BIOMEDICAL IMAGING (ISBI 2018), IEEE, 4 April 2018 (2018-04-04), pages 353 - 356, XP033348216, [retrieved on 20180523], DOI: 10.1109/ISBI.2018.8363591
- ZHENG XU ET AL: "Beyond Textures: Learning from Multi-domain Artistic Images for Arbitrary Style Transfer", ARXIV.ORG, CORNELL UNIVERSITY LIBRARY, 201 OLIN LIBRARY CORNELL UNIVERSITY ITHACA, NY 14853, 25 May 2018 (2018-05-25), XP080882286
- ANOOP CHERIAN ET AL: "Sem-GAN: Semantically-Consistent Image-to-Image Translation", ARXIV.ORG, CORNELL UNIVERSITY LIBRARY, 201 OLIN LIBRARY CORNELL UNIVERSITY ITHACA, NY 14853, 12 July 2018 (2018-07-12), XP081247481

## Beschreibung

Die Erfindung betrifft ein Verfahren zur Anpassung eines Bildeindrucks eines Bildes, insbesondere eines im Rahmen einer medizinischen Bildgebung ermittelten Bildes, umfassend die Schritte:
- Bereitstellen eines Bildes oder von Eingangsdaten, aus denen das Bild ermittelt wird,
- Vorgabe einer jeweiligen Sollbildeindrucksklasse für wenigstens ein direkt oder durch Vorgabe wenigstens eines Segmenttyps vorgegebenes Bildsegment des Bildes, wobei ein Zusammenhang zwischen den Bilddaten des jeweiligen Bildsegments und einer zugeordneten Bildeindrucksklasse durch einen Klassifikationsalgorithmus vorgegeben ist,
- Verändern des Bildes oder der Eingangsdaten durch einen Modifikationsalgorithmus, um die den hieraus resultierenden, veränderten Bilddaten des jeweiligen Bildsegments zugeordnete Bildeindrucksklasse der jeweiligen Sollbildeindrucksklasse anzugleichen, wobei wenigstens ein Modifikationsparameter des Modifikationsalgorithmus in Abhängigkeit des Klassifikationsalgorithmus vorgegeben ist oder wird,
- wobei für verschiedene Bildsegmente und/oder verschiedene Segmenttypen verschiedene Sollbildeindrucksklassen vorgegeben werden, wobei
- das Bild oder die Eingangsdaten durch einen Verarbeitungsalgorithmus, der von wenigstens einem Verarbeitungsparameter abhängt, aus Ursprungsdaten ermittelt sind oder werden, wobei die Bildeindrucksklasse des jeweiligen Bildsegments und/oder Segmenttyps von dem Verarbeitungsparameter abhängt,
- und der Verarbeitungsalgorithmus einen Rekonstruktionsalgorithmus zur Rekonstruktion von dreidimensionalen Volumendaten aus zweidimensionalen Ursprungsdaten, insbesondere aus Röntgenbildern, umfasst, wobei der Rekonstruktionsalgorithmus von dem Verarbeitungsparameter oder von wenigstens einem der Verarbeitungsparameter abhängt und/oder wobei das Bild oder die Eingangsdaten durch einen Abbildungsalgorithmus aus den Volumendaten generierte zweidimensionale Bilddaten sind, wobei der Abbildungsalgorithmus von dem Verarbeitungsparameter oder wenigstens einem der Verarbeitungsparameter abhängt.

Daneben betrifft die Erfindung eine Verarbeitungseinrichtung, ein Computerprogramm und einen elektronisch lesbaren Datenträger.

Im Rahmen einer medizinischen Bildgebung ermittelte Bilder werden durch Mediziner typischerweise im Rahmen einer Diagnose ausgewertet. Zudem werden entsprechende Bilder genutzt, um andere Personen, beispielsweise Kollegen oder Patienten, auf gewisse Merkmale in den Bildern hinzuweisen. Hierbei werden typischerweise nicht direkt erfasste Bilder visualisiert, sondern es erfolgt eine Vorverarbeitung der erfassten Daten. Dies ist insbesondere der Fall, wenn aus erfassten Daten, beispielsweise einzelnen Röntgenaufnahmen einer Computertomographie, zunächst dreidimensionale Volumendaten rekonstruiert werden und anschließend aus diesen zweidimensionale Bilder, beispielsweise Schnittbilder oder simulierte Röntgenaufnahmen, generiert werden. Es ist hierbei bekannt, dass der Bildeindruck des ermittelten Bildes beziehungsweise von einzelnen Merkmalen oder Segmenten im Bild von den im Rahmen der Bildermittlung gewählten Parametern abhängen kann. Beispielsweise können im Rahmen der Rekonstruktion eines Volumendatensatzes verschiedene Rekonstruktions-Kernel gewählt werden, die sich bezüglich der erreichbaren Ortsauflösung und dem Rauschpegel des Ergebnisses unterscheiden können. Je nach Einsatzzweck, also beispielsweise in Abhängigkeit davon, ob ein Bild dazu dienen soll, gewisse Informationen für einen Patienten zu visualisieren oder ob das Bild auf bestimmte Auffälligkeiten hin untersucht werden soll, und insbesondere in Abhängigkeit davon, in welchem Bildbereich welche Auffälligkeiten erkannt werden sollen, kann hierbei eine unterschiedliche Parametrisierung der Vorverarbeitung und somit ein unterschiedlicher resultierender Bildeindruck vorteilhaft sein. Zudem kann es sein, dass verschiedene Nutzer für den gleichen Zweck unterschiedliche Bildeindrücke wünschen, womit eine Verarbeitung der ursprünglich erfassten Daten mit anderen Parametern erforderlich ist, um den verschiedenen Nutzern jeweils ideale Bilder zur Verfügung stellen zu können.

Da es den Benutzern nicht notwendigerweise bewusst ist, welche Verarbeitungsparameter ideal für sie beziehungsweise eine gegebene Aufgabe sind, kann es erforderlich sein, mehrere Verarbeitungen von erfassten Daten durchzuführen, bis ein gewünschtes Ergebnis erreicht wird. Da beispielsweise bei einer Nutzung einer iterativen Rekonstruktion von Bilddaten ein relativ hoher Verarbeitungsaufwand resultiert, um ein neues Bild bereitzustellen, kann eine Änderung des gewünschten Bildeindrucks somit zu deutlichen Verzögerungen führen und somit den Arbeitsfluss eines Nutzers stören.

Aus dem Artikel YUBIN DENG ET AL.: "Aesthetic-Driven Image Enhancement by Adversarial Learning", ARXIV.ORG, CORNELL UNIVERSITY LIBRARY 201 OLIN LIBRARY CORNELL UNIVERSITY ITHACA, NY 14853, 17. Juli 2017 (2017-07-17), ist ein Ansatz zur ästhetikgetriebenen Bildverbesserung bekannt, der ein Generative Adversarial Network (GAN) nutzt. Ein Klassifikationsalgorithmus wird mit Bilddaten trainiert, denen jeweils ein Bildqualitätslabel zugeordnet ist, das angibt, ob die Bildqualität gut oder schlecht ist. Dieser wird als Diskriminator im Generative Adversarial Network genutzt, um einen Generator zu trainieren, der Bilder guter Qualität aus Eingangsbildern erzeugen soll.

In dem Artikel MANSOOR AWAIS ET AL: "Adversarial approach to diagnostic quality volumetric image enhancement", 2018 IEEE 15TH INTERNATIONAL SYMPOSIUM ON BIOMEDICAL IMAGING (ISBI 2018), IEEE, 4. April 2018 (2018-04-04), Seiten 353-356, wird ein Generative Adversarial Network genutzt, um Volumenbilder mit ausreichender Qualität für Diagnoseanwendungen im Bereich der Magnetresonanzbildgebung beziehungsweise der Computertomographie bereitzustellen. Als Eingangsdaten werden Bilddaten mit niedriger Auflösung genutzt. Ein Term der Kostenfunktion wird mit Hilfe eines Diskriminators bestimmt, der trainiert wird, um zwischen geschätzten Bildern und Bildern mit ausreichender Qualität für Diagnosezwecke zu unterscheiden.

Ein Ansatz, um einem Bild einen bestimmten künstlerischen Stil aufzuprägen, ist aus dem Artikel ZHENG XU ET AL: "Beyond Textures: Learning from Multi-domain Artistic Images for Arbitrary Style Transfer", ARXIV.ORG, CORNELL UNIVERSITY LIBRARY, 201 OLIN LIBRARY CORNELL UNIVERSITY ITHACA, NY 14853, 25. Mai 2018 (2018-05-25), bekannt. Hierbei wird ein Diskriminator eines Generative Adversarial Networks genutzt, der einerseits den artistischen Stil von Eingangsdaten klassifiziert und andererseits klassifiziert, ob es sich um reale oder gefälschte Eingangsdaten handelt.

Aus der Publikation ANOOP CHERIAN ET AL: "Sem-GAN: Semantically-Consistent lmage-tolmage Translation", ARXIV.ORG, CORNELL UNIVERSITY LIBRARY, 201 OLIN LIBRARY CORNELL UNIVERSITY ITHACA, NY 14853, 12. Juli 2018 (2018-07-12), XP081247481, befasst sich mit dem Problem der Bild-zu-Bild Übersetzung, das in der Zuordnung eines Bildes einer QuellDomäne zu einem Bild in einer Ziel-Domäne besteht. Um sicherzustellen, dass die übersetzten Bilder plausibel sind, wird gefordert, dass diese Zuordnung invertierbar ist. Diese Anforderung führt zu vielversprechenden Ergebnissen, wenn die Domänen unimodal sind. Multimodale Domänen jedoch, wie bei einer Bildsegmentierungsaufgabe, sind problematisch. Daher wird ein semantisch konsistentes GAN präsentiert, genannt Sem-GAN, in dem die Semantik durch die Klassenidentitäten von Bildsegmenten in der Quelldomäne definiert ist, wie sie von einem semantischen Segmentierungsalgorithmus erzeugt werden. Das vorgeschlagene GAN erzwingt, dass die übersetzten Bilder das Erscheinungsbild der Zieldomäne übernehmen, während ihre Identitäten aus der Quelldomäne weitestgehend erhalten bleiben. Es wird gezeigt, dass semantische Segmentierungsmodelle, die mit synthetischen, mit Sem-GAN übersetzten Bildern trainiert wurden, zu deutlich besseren Segmentierungsergebnissen führen als andere Varianten.

Der Erfindung liegt somit die Aufgabe zugrunde, den Arbeitsfluss bei einer Auswertung von Bilddaten durch einen Benutzer zu verbessern.

Die Aufgabe wird durch ein Verfahren der eingangs genannten Art gelöst.

Es wird somit vorgeschlagen, einen Bildeindruck bestimmter Segmente oder bestimmter Segmenttypen eines Bildes nicht dadurch zu ändern, dass Ursprungsdaten des Bildes erneut mit anderen Parametern verarbeitet werden, sondern stattdessen das Bild selbst durch eine Modifikationsalgorithmus zu modifizieren. Hierbei wird ein Modifikationsalgorithmus genutzt, der in Abhängigkeit eines Klassifikationsalgorithmus parametrisiert ist oder wird. Wie später noch detailliert erläutert werden wird, kann eine entsprechende Parametrisierung beispielsweise durch ein Verfahren des Maschinenlernens erfolgen. Prinzipiell wäre jedoch auch eine manuelle Parametrisierung unter Berücksichtigung eines vorgegebenen Klassifikationsalgorithmus möglich.

Das vorgeschlagene Vorgehen ermöglicht es, sobald ein entsprechender Modifikationsalgorithmus bereitsteht, den Bildeindruck bestimmter Segmente oder bestimmte Segmenttypen des Bildes bedarfsgerecht anzupassen. Hierbei kann eine solche Anpassung manuell gemäß bestimmter Anforderungen eines Nutzers erfolgen oder es kann in Abhängigkeit von bestimmten Parametern automatisiert eine Anpassung erfolgen, wie später noch genauer erläutert werden wird. Durch die schnelle Möglichkeit, einen Bildeindruck anzupassen, wird erreicht, dass ein Nutzer bedarfsgerecht schnell zwischen Bildeindrücken umschalten kann, um beispielsweise ohne eine Unterbrechung seines Arbeitsflusses einen Bildeindruck für das Bild aufzufinden, der ihn besonders gut bei seiner Aufgabe unterstützt. Das erfindungsgemäße Vorgehen ermöglicht es für unterschiedliche Segmente beziehungsweise Segmenttypen des Bildes unterschiedliche Bildeindrücke einzustellen. Beispielsweise kann erreicht werden, dass Merkmale in einigen Bildsegmenten nachgeschärft werden, während in anderen Bildsegmenten eine Reduktion eines Rauschens erfolgt. Dies wäre bei üblichen Verfahren sehr aufwendig, da Ursprungsdaten auf verschiedene Weise verarbeitet werden müssten und das resultierende Bild aus mehreren dieser Verarbeitungsergebnisse zusammengefügt werden müsste, um lokal unterschiedliche Bildeindrücke zu erreichen.

Der Klassifikationsalgorithmus wird im Verfahren selbst nicht notwendigerweise genutzt. Werden die Modifikationsparameter des Modifikationsalgorithmus in einem vorgelagerten Schritt, also nicht als Teil des Verfahrens selbst, ermittelt, ist es ausreichend, wenn der Klassifikationsalgorithmus in diesem vorgelagerten Schritt zur Verfügung steht. Der Klassifikationsalgorithmus kann, wie später noch genauer erläutert werden wird, insbesondere durch ein Verfahren des Maschinenlernens parametrisiert werden.

Der Modifikationsalgorithmus weist vorzugsweise eine Vielzahl von Modifikationsparametern auf, die im Rahmen des erfindungsgemäßen Verfahrens oder in einem vorgelagerten Schritt in Abhängigkeit des Klassifikationsalgorithmus ermittelt werden. Dies kann durch ein Maschinenlernen erfolgen. Hierbei können, wie später noch genauer erläutert werden wird, der Klassifikationsalgorithmus und der Modifikationsalgorithmus gemeinsam trainiert werden. Beispielsweise können diese Algorithmen im Rahmen des Trainings gemeinsam ein Generative Adversarial Network (GAN) bilden, wobei der Modifikationsalgorithmus als Generator und der Klassifikationsalgorithmus als Diskriminator arbeiten.

Die Bildeindrucksklasse kann beispielsweise eine Schärfe des Bildsegments oder eine Art der Darstellung, beispielsweise ob zusammenhängende Gebiete nur als Randlinien dargestellt werden, eine Helligkeit und/oder einen Kontrast, ob bestimmte Merkmale überhaupt dargestellt werden oder Ähnliches betreffen. Die Sollbildeindrucksklassen für einzelne Bildsegmente beziehungsweise Segmenttypen können direkt vorgegeben werden. Alternativ kann eine Darstellungsaufgabe, die für mehrere Bildsegmente beziehungsweise Segmenttypen jeweilige Sollbildeindrucksklassen vorgibt, vorgegeben werden. Beispielsweise kann vorgegeben werden, dass ein bestimmtes Organ scharf dargestellt werden soll, während andere Bildsegmente mit geringem Rauschen dargestellt werden sollen.

Ein Segmenttyp kann beispielsweise ein bestimmtes anatomisches Label sein, wobei durch den Modifikationsalgorithmus selbst oder durch eine vorgeschaltete Bildsegmentierung Bildsegmente erkannt werden können, die einem entsprechenden Label zugeordnet sind. Hierbei können Segmenttypen einzelne Organe, Knochen oder Ähnliches beschreiben, es ist jedoch auch möglich, dass ein Segmenttyp eine ganze Gruppe von Merkmalen beschreibt. Beispielsweise kann der Segmenttyp Knochen allen Bildsegmenten des Bildes zugeordnet sein, die Knochen zeigen oder Ähnliches.

Um eine Bildeindrucksklasse für ein bestimmtes Bildsegment zu verändern, kann im einfachsten Fall der Modifikationsalgorithmus ausschließlich auf Daten dieses Bildsegments angewandt werden. Dies kann beispielsweise dadurch erfolgen, dass das Bild vorangehend manuell oder automatisch durch einen, beispielsweise durch ein Maschinenlernen trainierten, Segmentieralgorithmus segmentiert wird. Bevorzugt erkennt der Modifikationsalgorithmus jedoch alle relevanten Bildbereiche, also beispielsweise Bildsegmente eines bestimmten Segmenttyps, selbst und modifiziert diese. Hierbei ist es nicht erforderlich, dass der Modifikationsalgorithmus explizit eine Segmentierung oder bestimmten Segmenten zugeordnete Bilddaten ausgibt. Vielmehr ist es ausreichend, wenn als Ergebnis erzielt wird, dass die Bildeindrucksklasse für das Entsprechende direkt oder über einen Segmenttyp vorgegebene Bildsegment an die für dieses Bildsegment beziehungsweise diesen Segmenttyp vorgegebene Sollbildeindrucksklasse angeglichen wird.

Ein Angleichen einer Bildeindrucksklasse eines Bildsegments kann dazu führen, dass die Bildeindrucksklasse des Bildsegments anschließend identisch zur Sollbildeindrucksklasse ist. Es ist jedoch auch möglich, dass die Bildeindrucksklassen auf eine bestimmte Weise geordnet sind. Dabei kann eine eindimensionale Ordnung, also beispielsweise eine Ordnung nach einem erreichten Schärfegrad des Bildes beziehungsweise des Bildsegments, erfolgen, es ist jedoch auch eine mehrdimensionale Ordnung möglich, also beispielsweise eine Ordnung nach Schärfegrad und Kontrast. Unter einem Angleichen kann hierbei verstanden werden, dass die Bildeindrucksklasse des Segments nach Anwendung des Modifikationsalgorithmus näher an der Sollbildeindrucksklasse liegt als sie dies für das ursprüngliche Bildsegment tat.

Die Bilddaten sind vorzugsweise zweidimensionale Bilddaten. Insbesondere kann es sich um zweidimensionale Bilddaten handeln, die aus zweidimensionalen Volumendaten generiert sind, beispielsweise um Schnittbilder oder künstliche Projektionen.

Bei den Eingangsdaten handelt es sich vorzugsweise um mehrere zweidimensionale Bilder, die kombiniert werden, um das Bild bereitzustellen. Beispielsweise können die Eingangsdaten ein im Rahmen einer Angiographie angefertigtes Maskenbild und ein Bild mit Kontrastmittel sein und das Bild kann durch Subtraktion dieser Eingangsbilder voneinander ermittelt werden. Zur Veränderung des Bildes kann das resultierende Bild modifiziert werden. Es ist jedoch auch möglich, die Eingangsdaten, beispielsweise das Kontrastmittelbild beziehungsweise das Maskenbild, separate durch den Modifikationsalgorithmus zu modifizieren, um hierüber indirekt das Bild zu verändern.

Der Modifikationsparameter kann durch ein Verfahren des Maschinenlernens ermittelt sein oder werden. Hierbei kann im einfachsten Fall der Klassifikationsalgorithmus fest vorgegeben sein. Beispielsweise kann der Klassifikationsalgorithmus ein vorangehend trainiertes Convolutional Neural Network sein. Mit Hilfe des Klassifikationsalgorithmus kann ein unüberwachtes Lernen der Modifikationsparameter erfolgen, das heißt es ist nicht erforderlich, zu erreichende Ergebnisse, beispielsweise in Form von bereits entsprechend modifizierten Bildern, vorzugeben. Zum Training des Modifikationsalgorithmus kann es ausreichend sein, einen Trainingsdatensatz vorzugeben, der ausschließlich die zu modifizierenden Bilder und Anweisungen, wie diese modifiziert werden sollen, umfasst.

Die Modifikationsparameter des Modifikationsalgorithmus, also beispielsweise Gewichtungsfaktoren einzelner Neuronen, wenn ein neuronales Netz als Modifikationsalgorithmus trainiert wird, können zunächst beliebig, beispielsweise zufällig, initialisiert werden. Als Eingangsdaten werden dem Modifikationsalgorithmus beispielsweise ein jeweiliges Bild und eine jeweilige gewünschte Modifikation, also eine Sollbildeindrucksklasse für bestimmte Segmente oder Segmenttypen, vorgegeben. Mit Hilfe des bekannten Klassifikationsalgorithmus kann nach der Modifikation des jeweiligen Bildes geprüft werden, ob diese erfolgreich war beziehungsweise wie erfolgreich diese war. Dies kann anschließend genutzt werden, um die Modifikationsparameter des Modifikationsalgorithmus zu modifizieren. Der Modifikationsalgorithmus kann insbesondere ein generatives Netzwerk beziehungsweise ein Deconvolution-Netzwerk sein. Derartige neuronale Netze sind besonders geeignet, bestimmte Bilddaten zu generieren beziehungsweise zu modifizieren.

Das Training des Modifikationsalgorithmus, also die Ermittlung der Modifikationsparameter, kann ein Verfahrensschritt des erfindungsgemäßen Verfahrens sein. Es ist jedoch auch möglich, dieses Training als separates Verfahren vorzulagern. Insofern betrifft die Erfindung auch ein Verfahren zum Training eines Modifikationsalgorithmus, der zum Verändern eines Bildes oder von Segmenten des Bildes zur Veränderung eines Bildeindrucks dient.

Zum Training des Modifikationsalgorithmus können vorsegmentierte Bilder genutzt werden, die durch den Modifikationsalgorithmus modifiziert werden sollen. Die Segmentierung kann manuell oder durch einen, insbesondere durch ein vorangehendes Maschinenlernen trainierten, Segmentieralgorithmus erfolgen. Hierbei können den einzelnen Segmenten beziehungsweise Gruppen von Segmenten Segmenttypen zugeordnet sein. Prinzipiell ist es möglich, die Segmentierung betreffende Segmentierungsinformationen auch dem Modifikationsalgorithmus als Eingangsdaten zur Verfügung zu stellen. Dies kann beispielsweise vorteilhaft sein, wenn der Modifikationsalgorithmus dazu trainiert werden soll, vorsegmentierte Bilder zu modifizieren.

Es kann jedoch auch vorteilhaft sein, die Segmentierungsinformation nicht im Rahmen des Modifikationsalgorithmus auszuwerten, sondern ausschließlich dazu zu nutzen, für die einzelnen vorgegebenen Segmente der veränderten Bilddaten durch den Klassifikationsalgorithmus Bildeindrucksklassen zu ermitteln und diese mit der Sollbildeindrucksklasse für das entsprechende Segment beziehungsweise für den entsprechenden Segmenttyp zu vergleichen und in Abhängigkeit dieses Vergleichs die Modifikationsparameter anzupassen. Der Modifikationsalgorithmus kann in diesem Fall als Eingangsdaten zusätzlich zum Bild Paare von Segmenttypen und zu den Segmenttypen vorgegebenen Sollbildeindrucksklassen erhalten. Der Modifikationsalgorithmus wird somit dazu trainiert, selbständig zu erkennen, welche Bildbereiche wie modifiziert werden müssen, um eine Angleichung der Bildeindrucksklasse von Bildsegmenten eines bestimmten Segmenttyps an eine Sollbildeindrucksklasse zu erreichen. Hierdurch können beispielsweise Artefakte vermieden werden, die resultieren könnten, wenn verschiedene Bildsegmente des Bildes auf unterschiedliche Weise modifiziert werden, um unterschiedliche Bildeindrucksklassen für diese Segmente zu erreichen.

Wenigstens ein Klassifikationsparameter des Klassifikationsalgorithmus und der Modifikationsparameter können gemeinsam durch ein Verfahren des Maschinenlernens ermittelt sein oder werden. Hierbei wird zunächst insbesondere ein überwachtes Training des Klassifikationsalgorithmus durchgeführt. Beispielsweise kann ein Trainingsdatensatz hierfür mehrere Bilder umfassen, wobei den Bildern beziehungsweise den einzelnen Segmenten oder Segmenttypen der Bilder, beispielsweise durch eine vorangehende manuelle Klassifizierung, jeweilige Bildeindrucksklassen zugeordnet sind. Der Klassifikationsalgorithmus kann beispielsweise ein neuronales Netz, beispielsweise ein Convolutional Neural Network, sein. Ansätze zum überwachten Lernen von Klassifikationsalgorithmen sind grundsätzlich bekannt und sollen daher nicht detailliert erläutert werden. Beispielsweise kann eine Fehlerrückführung genutzt werden, um eine Abweichung zwischen einer durch den Klassifikationsalgorithmus bestimmten Bildeindrucksklasse und der mit dem Trainingsdatensatz vorgegebenen Bildeindrucksklasse zu minimieren.

Ein anschließendes gemeinsames Training von Klassifizierungsalgorithmus und Modifikationsalgorithmus ermöglicht eine weitere kontinuierliche Verbesserung des Klassifikationsalgorithmus und somit letztlich auch des Modifikationsalgorithmus. Hierbei können der Klassifikationsalgorithmus und der Modifikationsalgorithmus abwechselnd trainiert werden oder es kann ein gemeinsames Training erfolgen.

Im Rahmen des Maschinenlernens kann ein Lernalgorithmus zugleich oder abwechseln versuchen, den Modifikationsparameter derart zu wählen, dass bei einem Anwenden des Klassifikationsalgorithmus auf ein direkt oder durch Vorgabe wenigstens eines Segmenttyps vorgegebenes Bildsegment eines Ergebnisbildes, das durch Anwenden des Modifikationsalgorithmus auf ein Eingangsbild ermittelt wird, eine vorgegebene Sollbildeindrucksklasse ermittelt wird, und den Klassifikationsparameter derart zu wählen, dass bei einem Anwenden des Klassifikationsalgorithmus auf das wenigstens eine Bildsegment des Ergebnisbildes und auf ein dem Bildsegment des Ergebnisbildes zugeordnetes Bildsegment des Eingangsbildes die gleiche Bildeindrucksklasse ermittelt wird.

Anders ausgedrückt wird zugleich versucht, den Modifikationsalgorithmus derart zu trainieren, dass das Segment bei einem anschließenden Anwenden des Klassifikationsalgorithmus die vorgegebene Sollbildeindrucksklasse aufweist, während der Klassifikationsalgorithmus darauf trainiert wird, eine solche Manipulation zu erkennen. Ein möglicher Ansatz hierfür ist es, ein Generatives Adversarial Network (GNA) zu trainieren, in dem der Modifikationsalgorithmus als generatives Netzwerk genutzt wird und der Klassifikationsalgorithmus als Diskriminator. Durch den beschriebenen Lernansatz wird insbesondere erreicht, dass abgesehen vom vorangehenden Training des Klassifikationsalgorithmus das Training des Modifikationsalgorithmus unüberwacht erfolgen kann, das heißt, dass keine Sollergebnisse für den Modifikationsalgorithmus vorgegeben werden müssen, da statt einem Vergleich mit einem Sollergebnis ein Lernen mit Hilfe des Klassifikationsalgorithmus erfolgt.

Je nach Anzahl der zu unterscheidenden Bildeindrucksklassifikationen kann es auch vorteilhaft sein, mehrere parallele oder kombinierte neuronale Netze zu nutzen, um die Klassifikationsparameter und Modifikationsparameter zu bestimmen.

Im Rahmen des Trainings können auch für mehrere Segmente oder Segmenttypen jeweilige Sollbildeindrucksklassen vorgegeben werden. In diesem Fall gilt die obige Erläuterung für jedes Paar aus Bildsegment des Ergebnisbildes und zugeordnetes Bildsegment des Eingangsbildes.

In dem erfindungsgemäßen Verfahren wird oder ist das Bild oder werden oder sind die Eingangsdaten durch einen Verarbeitungsalgorithmus, der von wenigstens einem Verarbeitungsparameter abhängt, aus Ursprungsdaten ermittelt, wobei die Bildeindrucksklasse des jeweiligen Bildsegments und/oder Segmenttyps von dem Verarbeitungsparameter abhängt. Die Verarbeitung der Ursprungsdaten ist hierbei Teil des erfindungsgemäßen Verfahrens, so dass für das erfindungsgemäße Verfahren das Bild beziehungsweise die Eingangsdaten bereitgestellt werden. Hierbei kann der Klassifikationsalgorithmus derart vorgegeben werden beziehungsweise der wenigstens eine Klassifikationsparameter des Klassifikationsalgorithmus derart ermittelt werden, dass durch den Klassifikationsalgorithmus ermittelte Bildeindrucksklassen von dem jeweils genutzten Verarbeitungsparameter abhängen. Dies kann beispielsweise durch ein Maschinenlernen erfolgen. Beispielsweise kann ein überwachtes Lernen implementiert werden, indem aus vorzugsweise mehreren vorgegebenen Ursprungsdatensätzen jeweils mit verschiedenen Verarbeitungsparametern das Bild beziehungsweise die Eingangsdaten generiert werden. Hierbei können Sätzen von Verarbeitungsparametern, beispielsweise aufgrund eines Vorwissens über das Wirken dieser Verarbeitungsparameter, fest bestimmten Bildeindrucksklassen zugeordnet werden. Somit kann der Trainingsdatensatz zu jedem Bild beziehungsweise zu jedem Eingangsdatensatz eine den Verarbeitungsparametern zugeordnete Bildeindrucksklassifikation umfassen. Ist der Klassifikationsalgorithmus beispielsweise als neuronales Netz implementiert, so kann das Lernen anschließend durch einen Vergleich der ermittelten Bildeindrucksklassifikation und der im Trainingsdatensatz gespeicherten Bildeindrucksklassifikation erfolgen und Klassifikationsparameter des Klassifikationsalgorithmus können beispielsweise durch eine Fehlerrückführung angepasst werden.

Der Verarbeitungsparameter kann beispielsweise einen Einfluss auf die Bildschärfe oder ein Bildrauschen im Bild oder in bestimmten Segmenten des Bildes haben. Dies kann beispielsweise der Fall sein, wenn der Verarbeitungsalgorithmus in Abhängigkeit des Verarbeitungsparameters einen Faltungs-Kernel oder Ähnliches anpasst. Die durch den Klassifikationsalgorithmus ermittelten Bildeindrucksklassen können in diesem Fall beispielsweise angeben, wie scharf oder weich gezeichnet ein bestimmtes Segment des Bildes ist. Der Modifikationsalgorithmus kann in diesem Fall dazu dienen, das Bild nachträglich entsprechend zu glätten beziehungsweise zu schärfen.

In dem erfindungsgemäßen Verfahren umfasst der Verarbeitungsalgorithmus einen Rekonstruktionsalgorithmus zur Rekonstruktion von dreidimensionalen Volumendaten aus zweidimensionalen Ursprungsdaten, insbesondere aus Röntgenbildern, wobei der Rekonstruktionsalgorithmus von dem Verarbeitungsparameter oder von wenigstens einem der Verarbeitungsparameter abhängt und/oder wobei das Bild oder die Eingangsdaten durch einen Abbildungsalgorithmus aus den Volumendaten generierte zweidimensionale Bilddaten sind, wobei der Abbildungsalgorithmus von dem und/oder wenigstens einem der Verarbeitungsparameter abhängt. Beispielsweise können die dreidimensionalen Volumendaten im Rahmen einer Computertomographie aus zweidimensionalen Röntgenbildern generiert werden, beispielsweise durch eine gefilterte Rückprojektion, eine iterative Rekonstruktion oder Ähnliches. Bei den Bilddaten oder Eingangsdaten kann es sich beispielsweise um Schichtbilder oder aus den Volumendaten generierte synthetische Röntgenbilder handeln.

Der Rekonstruktionsparameter kann beispielsweise eine Auswahl des genutzten Rekonstruktionskernels beziehungsweise dessen Parametrisierung betreffen. Beispielsweise kann ein Rekonstruktionskernel so gewählt werden, dass die Volumendaten Hounsfield-Einheiten angeben oder es kann ein Rekonstruktionskernel gewählt werden, der Kanten hervorhebt.

In dem erfindungsgemäßen Verfahren werden für verschiedene Bildsegmente und/oder verschiedene Segmenttypen verschiedene Sollbildeindrucksklassen vorgegeben. Beispielsweise kann es gewünscht sein, dass in einem bestimmten relevanten Bereich, beispielsweise im Bereich eines bestimmten Organs, eine besonders scharfe Darstellung erfolgt oder dass Kanten hervorgehoben werden, während im umliegenden Hintergrundbereich eher eine Rauschunterdrückung gewünscht ist, auch dann, wenn dies unter Umständen zu einer Reduktion der Ortsauflösung führen kann. Es kann auch gewünscht sein, bestimmte für eine aktuelle Visualisierung wenig relevante Segmenttypen nicht oder beispielsweise mit geringerer Helligkeit oder geringem Kontrast darzustellen, damit relevante Merkmale schneller und leichter zu erkennen sind. Hierbei können bedarfsgerecht automatisch oder manuell verschiedene Darstellungen und somit entsprechende Sätze von Sollbildeindrucksklassen für die verschiedenen Bildsegmente beziehungsweise Segmenttypen gewählt werden. Beispielsweise kann es durch entsprechende Wahl der Sollbildeindrucksklassen erreicht werden, dass auf Basis des gleichen Bilds beziehungsweise der gleichen Eingangsdaten bedarfsgerecht entweder eher Knochen oder eher das Gefäßsystem hervorgehoben werden.

Die Sollbildeindrucksklasse und/oder das wenigstens eine Bildsegment und/oder der wenigstens eine Segmenttyp, für das oder den die Sollbildeindrucksklasse vorgegeben wird, können in Abhängigkeit einer Bedieneingabe eines Benutzers und/oder einer den Benutzer identifizierenden Benutzerinformation und/oder des Bildes und/oder der Eingangsdaten und/oder der Ursprungsdaten und/oder einer die Bildgebung des Bildes und/oder einen in dem Bild abgebildeten Patienten betreffenden Zusatzinformation vorgegeben werden. Dies kann dazu dienen, beispielsweise bereits beim Öffnen eines Bildes durch einen Benutzer, automatisch das Bild so zu modifizieren, dass ein für einen bestimmten Benutzer beziehungsweise für eine bestimmte Aufgabe geeigneter Bildeindruck entsteht. Es ist jedoch auch möglich, dass die genannten Parameter nur genutzt werden, um einen Benutzer bei einer Auswahl einer geeigneten Modifikation zu unterstützen. Beispielsweise können in Abhängigkeit dieser Parameter nur bestimmte Modifikationen vorgeschlagen oder beispielsweise die Reihenfolge der vorgeschlagenen Modifikationen angepasst werden. Diese Ansätze können auch kombiniert werden. Beispielsweise kann zunächst eine automatische Modifikation des Bildes erfolgen und der Benutzer kann bedarfsgerecht zu anderen Modifikationen umschalten, wobei ihm hierfür eine geeignete Auswahl angeboten wird.

Eine Vorgabe der Parameter in Abhängigkeit der Benutzerinformation kann vorteilhaft erfolgen, um ein dargestelltes modifiziertes Bild an die Vorlieben eines Benutzers anzupassen. Diese können beispielsweise kontinuierlich erlernt werden. Beispielsweise kann es anfangs erforderlich sein, dass ein dem System noch nicht bekannter Benutzer manuell auswählen muss, wie ein Bild modifiziert werden soll, also insbesondere welche Segmente oder Segmenttypen welche Sollbildeindrucksklassen aufweisen sollen. Nach einer oder mehreren dieser Eingaben kann das System, beispielsweise durch ein Verfahren des Maschinenlernens oder durch eine statistische Auswertung der Eingaben, insbesondere unter Berücksichtigung der jeweiligen Nutzungssituation, in der eine entsprechende Eingabe erfolgte, Vorlieben des Benutzers erlernen und anschließend beispielsweise gemäß dieser Vorlieben bereits automatisch eine Modifikation wählen beziehungsweise die vorgeschlagenen Modifikationen einschränken. Hierbei kann auch nach dem Erlernen der Benutzervorlieben das Lernen weiter fortgesetzt werden, also beispielsweise eine Feedbackschleife implementiert werden, um zu erkennen, wenn der Benutzer in bestimmten Situationen von der angenommenen optimalen Modifikation zu einer anderen Modifikation wechselt, um beispielsweise geänderte Benutzervorlieben zu erkennen.

Die Benutzerinformation kann beispielsweise ermittelt werden, indem sich ein Benutzer dem System gegenüber identifiziert, beispielsweise durch eine Eingabe einer Benutzerkennung, eine Key-Card oder Ähnliches. Es ist jedoch auch möglich, eine automatisierte Benutzererkennung durchzuführen. Hierzu können beispielsweise Eingabemuster, beispielsweise eine zeitliche Abfolge von Tastatureingaben, Mausgesten, die Auswahl bestimmter Programmoptionen und Ähnliches ausgewertet werden. So kann beispielsweise ein Algorithmus des Maschinenlernens genutzt werden, um Nutzern anhand ihrer Handlungen am System zu identifizieren.

Eine Abhängigkeit der Sollbildeindrucksklasse und/oder des Bildsegments und/oder des Segmenttyps von dem Bild, den Eingangsdaten und/oder den Ursprungsdaten kann vorteilhaft sein, wenn beispielsweise eine automatische Erkennung von Merkmalen in diesen Daten erfolgt. Beispielsweise kann erkannt werden, dass in einem gewissen Bildsegment Merkmale vorhanden sind, die auf eine Läsion, einen Tumor oder eine andere Anomalie hindeuten. Durch Wahl einer entsprechenden Solleindrucksklasse für dieses Bildsegment beziehungsweise anderen entsprechende Manipulationen des Bildes kann erreicht werden, dass entsprechende unter Umständen hochrelevante Merkmale für einen Benutzer deutlich und einfach zu erkennen sind.

Als Parameter der Bildgebung können beispielsweise genutzte Belichtungszeiten, Strahlungsintensitäten beziehungsweise Röhrenströme, ein zeitlicher Verlauf der Erfassung der Röntgenaufnahmen, Informationen, dass eine Unterabtastung erfolgte, oder Ähnliches berücksichtigt werden. Parameter der Bildgebung können einen starken Einfluss auf die Darstellung bestimmter Merkmale in Bildern haben, womit es zweckmäßig sein kann, in Abhängigkeit von Bildgebungsparametern anzupassen, wie ein Bild modifiziert werden soll, also welche Solleindrucksklasse für das Bild beziehungsweise für bestimmte Bildsegmente oder Segmenttypen des Bildes gewählt wird.

Informationen die den Patienten betreffen können beispielsweise Informationen aus einer Patientenakte sein. Aus diesen kann sich beispielsweise ergeben, welchem Zweck die Bildaufnahme diente beziehungsweise welche Darstellungs- beziehungsweise Diagnoseaufgabe durch das Bild gelöst werden soll. So kann es für die Wahl eines zweckmäßigen Bildeindrucks hochrelevant sein, ob für eine aktuelle Diagnoseaufgabe eher ein Gefäßsystem oder eher Knochen, die in dem Bild dargestellt sind, relevant sind.

Die genannte Liste von Parametern, von denen die Auswahl der Sollbildeindrucksklasse beziehungsweise der modifizierten Bildsegmente oder Segmenttypen abhängt, ist nicht abschließend. Beispielsweise könnten zusätzlich vorangehende Bedieneingaben des Benutzers, insbesondere während der Betrachtung des gleichen Bildes, berücksichtigt werden, um weitere Rückschlüsse auf die Wünsche des Benutzers zu ziehen oder es könnte berücksichtigt werden, ob eine Visualisierung beispielsweise in einem Sprechzimmer, also unter Umständen für einen Patienten, an einem Messgerät oder an einem Bürorechner erfolgt.

Die Vorgabe der Sollbildeindrucksklasse und/oder des wenigstens einen Bildsegments und/oder Segmenttyps, für das oder den die Sollbildeindrucksklasse vorgegeben wird, und/oder eine Vorgabe von durch den oder einen Benutzer wählbaren oder diesem empfohlenen Sollbildeindrücken und/oder Bildsegmenten und/oder Segmenttypen, für die die Sollbildeindrücke wählbar sind oder empfohlen oder vorgegeben werden, kann durch einen Vorgabealgorithmus erfolgen, der durch ein Maschinenlernen parametrisiert ist oder wird. Das Training kann erfolgen, indem potentiell für eine Auswahl relevante Parameter, beispielsweise die vorangehend diskutierten Parameter, für eine bestimmte Benutzungssituation und eine in dieser Benutzungssituation erfolgte Auswahl der Solleindrucksklasse oder -klassen beziehungsweise der zu verändernden Segmente beziehungsweise Segmenttypen durch den Benutzer als Trainingsdaten genutzt werden. Der Vorgabealgorithmus kann durch ein überwachtes Lernen trainiert werden, wobei die manuelle Auswahl das Sollergebnis des Vorgabealgorithmus vorgibt und die erfassten relevanten Parameter die Eingangsgrößen. Hierbei kann nach einer Anlernphase des Vorgabealgorithmus eine weitere Überwachung des Benutzers erfolgen, um beispielsweise Änderungen im Benutzerverhalten zu erkennen.

Der Modifikationsalgorithmus kann dazu eingerichtet sein, in Abhängigkeit einer für das Bild vorgegebenen Darstellungsaufgabe automatisch Bildsegmente und/oder Segmenttypen und jeweils zugeordnete Sollbildeindrucksklassen vorzugeben. Dies kann insbesondere zweckmäßig sein, wenn eine manuelle Auswahl der Bildmodifikation durch einen Benutzer erfolgen soll und zumindest für Teile der möglichen Darstellungsaufgaben unterschiedliche Sollbildeindrucksklassen für unterschiedliche Bildsegmente beziehungsweise Segmenttypen verwendet werden sollen. Es ist in diesem Fall vorteilhaft, eine Art Makro zu definieren, das einem Benutzer ermöglicht, durch Auswahl einer bestimmten Darstellungsaufgabe komplexe Modifikationen am Bild durchzuführen.

Im Rahmen einer bestimmten Darstellungsaufgabe, jedoch auch unabhängig davon, kann durch Vorgabe entsprechender Sollbildeindrucksklassen für die entsprechenden Segmente beziehungsweise Segmenttypen beispielsweise erreicht werden, dass bestimmte vorhandene Merkmale beziehungsweise Segmente, die diese umfassen, mit geringer Helligkeit oder geringem Kontrast oder nicht oder nur schematisch dargestellt werden. Eine Nichtdarstellung bestimmter Merkmale kann beispielsweise dadurch erfolgen, dass Teile der Segmente durch eine einfarbige Fläche oder ein bestimmtes Muster ersetzt werden. Dies kann beispielsweise dann vorteilhaft sein, wenn ein bestimmter Sachverhalt für einen Patienten visualisiert werden soll und diesbezüglich nicht relevante Merkmale ausgeblendet werden sollen.

Eine schematische Darstellung kann beispielsweise dadurch erfolgen, dass in gewissen Bildsegmenten nur Randlinien von erfassten Merkmalen dargestellt werden. Dies kann beispielsweise dadurch erreicht werden, dass in diesen Segmenten ein Gradient gebildet wird und anschließend ein Grenzwertvergleich erfolgt. Optional können auch morphologische Operationen genutzt werden, um geschlossene Randlinien zu bilden. Selbstverständlich sind auch noch weitergehende Abstraktionen möglich, beispielsweise, dass Gefäße ausschließlich durch ihre Mittellinie dargestellt werden oder Ähnliches. Wird das Bild aus Eingangsdaten, die mehrere Bilder umfassen, ermittelt, beispielsweise für eine digitale Subtraktionsangiographie, ist es auch möglich, gewisse Segmente oder Segmenttypen transparent darzustellen. Beispielsweise kann je nach gewählter Darstellungsaufgabe beziehungsweise Sollbildeindrucksklasse der Hintergrund einer Angiographieaufnahme, also beispielsweise Knochen und/oder andere Organe, vollständig ausgeblendet werden oder zur Orientierung des Benutzers mit geringem Kontrast beziehungsweise geringer Helligkeit dargestellt werden.

Ein mögliches Beispiel für eine Darstellungsaufgabe ist es, Knochen aus dem Bild zu entfernen. Hierbei können die Knochen vorsegmentiert sein oder sie können durch den Modifikationsalgorithmus selbst segmentiert werden. In diesem Fall kann beispielsweise die Darstellungsaufgabe "Knochen entfernen" vorgeben, dass für den Segmenttyp Knochen der Inhalt der entsprechenden Segmente nicht, mit geringerer Helligkeit beziehungsweise geringerem Kontrast oder nur schematisch dargestellt wird.

Das Bild oder die veränderten Bilddaten können für den oder einen Benutzer dargestellt werden, wobei wenigstens ein Bildbereich markiert wird, der ein Merkmal umfasst, dessen Darstellung von der Bildeindrucksklassifikation des Bildes oder wenigstens eines Segments des Bildes oder von der Darstellungsaufgabe abhängt. Bestimmte in dem Bild abgebildete Merkmale können in dem Bild selbst oder bei bestimmten modifizierten Fassungen des Bildes nur schwer erkennbar sein. Dies kann unter Umständen auch gewünscht sein, da es die Erkennung anderer Merkmale verbessern kann. Zugleich können entsprechende Merkmale dennoch für eine aktuelle Diagnoseaufgabe oder Ähnliches relevant sein. Es kann daher vorteilhaft sein, insbesondere Bildbereiche, in denen sich in einer aktuellen Darstellung schwer oder nicht erkennbare Merkmale befinden, zu markieren, um einen Benutzer darauf hinzuweisen, dass durch Wahl anderer Sollbildeindrucksklassen beziehungsweise eine andere Darstellungsaufgabe dieses Merkmal darstellbar beziehungsweise deutlicher darstellbar ist.

Insbesondere kann vorgesehen sein, dass der Benutzer markierte Bildbereiche durch eine Bedieneingabe wählen kann. Beispielsweise kann das Bild auf einem Touchscreen dargestellt werden und entsprechende Bildbereiche können durch einen Benutzer berührt werden, sie können mit einer Maus auswählbar sein oder Ähnliches. Bei einer Anwahl des markierten Bildbereichs kann automatisch oder nach Rückfrage bei einem Benutzer eine andere Darstellungsaufgabe oder für das gesamte Bild oder zumindest ein Segment eine andere Sollbildeindrucksklasse vorgegeben werden, um durch eine erneute Modifikation des Bildes eine veränderte, insbesondere deutlichere, Darstellung des Merkmals zu erreichen.

Das Bild kann durch eine überlagerte Darstellung, insbesondere durch eine Subtraktion, von durch die Eingangsdaten beschriebenen Quellbildern ermittelt werden, wobei der Modifikationsalgorithmus die Quellbilder als Eingangsdaten verarbeitet. Insbesondere kann das Bild eine digitale Subtraktionsangiographie darstellen, bei der ein ohne Kontrastmittel aufgenommenes Maskenbild von einem mit Kontrastmittel aufgenommenen Kontrastmittelbild abgezogen wird, um eine deutlichere Hervorhebung des Gefäßsystems des Patienten zu erreichen. Hierbei kann insbesondere eine CT-Angiographie genutzt werden, bei der eine CT-Aufnahme mit und ohne Kontrastmittel erfolgen kann, so dass das Kontrastmittelbild und das Maskenbild insbesondere Schnittbilder eines jeweiligen entsprechenden Volumendatenersatzes sein können. Hierbei wäre es zwar prinzipiell möglich, den Modifikationsalgorithmus auf das aus der Überlagerung resultierende Bild anzuwenden. Die Verarbeitung der Quellbilder als Eingangsdaten ermöglicht es jedoch, die Quellbilder separat voneinander zu modifizieren beziehungsweise die Art der Überlagerung anzupassen. So kann es beispielsweise für eine gewisse Darstellungsaufgabe beziehungsweise einen gewissen Bildeindruck gewünscht sein, dass die in der digitalen Subtraktionsangiographie typischerweise im Wesentlichen vollständig ausgeblendeten Organe schemenhaft oder mit vollem Kontrast eingeblendete werden. Es kann auch möglich sein, das Subtraktionsbild, das Ausschließlich das Gefäßsystem zeigt, anschließend erneut mit dem Maskenbild zu überlagern, wobei eine unterschiedliche Modifikation des Maskenbilds und des Subtraktionsbildes erfolgt, also beispielsweise das Subtraktionsbild nachgeschärft wird, während das Maskenbild weichgezeichnet wird oder Ähnliches. Es kann somit eine bedarfsgerechte, flexibel anpassbare Darstellung für einen Benutzer bereitgestellt werden.

Eine Anpassung einer überlagerten Darstellung, wobei die Quellenbilder als Eingangsdaten des Modifikationsalgorithmus genutzt werden, kann beispielsweise auch dazu genutzt werden, Informationen aus verschiedenen parallelen Schichten eines Volumendatensatzes gemeinsam in einem Bild darzustellen. In Abhängigkeit der Darstellungsaufgabe beziehungsweise der Sollbildeindrucksklasse für das gesamte Bild oder das einzelne Bildsegment oder den einzelnen Segmenttyp kann der Modifikationsalgorithmus die Überlagerung beispielsweise derart beeinflussen, dass in bestimmten Segmenten oder Segmenttypen ausschließlich Informationen einer der Schichten dargestellt werden, oder dass eine Überlagerung erfolgt, so dass Informationen aus einer der Schichten als eine Art transparente Schicht über den Informationen der anderen Schicht liegen.

Wie bereits erläutert kann die Ermittlung des wenigstens einen Klassifikationsparameters des Klassifikationsalgorithmus und/oder die Ermittlung des wenigstens einen Modifikationsparameters des Modifikationsalgorithmus unabhängig von dem erfindungsgemäßen Verfahren als vorgelagerter Schritt erfolgen. Der Klassifikationsparameter und/oder der Modifikationsparameter können hierbei durch ein jeweiliges Verfahren des Maschinenlernens oder insbesondere gemeinsam durch ein Verfahren des Maschinenlernens ermittelt werden.

Daher betrifft die Erfindung auch ein Verfahren zur Ermittlung eines Klassifikationsparameters eines Klassifikationsalgorithmus, der dazu dient, Bilddaten eines Bildes und/oder eines jeweiligen Bildsegments eine Bildeindrucksklasse zuzuordnen, durch ein Verfahren des Maschinenlernens. Dies wurde bereits vorangehend im Detail erläutert.

Zudem betrifft die Erfindung somit ein Verfahren zur Ermittlung wenigstens eines Modifikationsparameters eines Modifikationsalgorithmus, der dazu dient, ein Bild oder Eingangsdaten, aus denen das Bild ermittelt wird, derart zu verändern, dass eine Bildeindrucksklasse, die hieraus resultierenden, veränderten Bilddaten des Bildes oder eines jeweiligen Bildsegments des Bildes durch einen Klassifikationsalgorithmus zugeordnet ist, einer vorgegebenen Sollbildeindrucksklasse angeglichen wird, wobei der Modifikationsparameter durch ein Verfahren des Maschinenlernens ermittelt wird. Insbesondere können der Modifikationsparameter und ein Klassifikationsparameter, der den Klassifikationsalgorithmus parametrisiert, gemeinsam durch ein Verfahren des Maschinenlernens ermittelt werden. Die Ermittlung des Modifikationsparameters beziehungsweise die gemeinsame Ermittlung von Modifikationsparameter und Klassifikationsparameter wurden bereits vorangehend ausführlich erläutert.

Somit betrifft die Erfindung auch einen elektronisch lesbaren Datenträger, auf dem durch das vorangehend erläuterten Verfahren ermittelte oder ermittelbare Klassifikationsparameter und/oder Modifikationsparameter und/oder eine Implementation eines durch diese Klassifikationsparameter parametrisierten Klassifikationsalgorithmus und/oder eines durch diese Modifikationsparameter parametrisierten Modifikationsalgorithmus gespeichert sind.

Neben dem erfindungsgemäßen Verfahren betrifft die Erfindung eine Verarbeitungseinrichtung zur Verarbeitung von Bildern, die zur Durchführung des erfindungsgemäßen Verfahrens eingerichtet ist. Die Verarbeitungseinrichtung kann insbesondere in eine Röntgeneinrichtung, beispielsweise in einen Computertomographen, integriert sein beziehungsweise Teil eines Röntgensystems sein, das insbesondere eine Röntgeneinrichtung zur Erfassung von Röntgenbildern, insbesondere einen Computertomographen, umfasst. Es ist jedoch auch möglich, dass die Verarbeitungseinrichtung ein entsprechend programmierter Arbeitsplatzrechner oder Server ist. Ein solcher Server kann beispielsweise lokal im gleichen Gebäude wie eine genutzte Bilderfassungseinrichtung, insbesondere eine Röntgeneinrichtung, jedoch beabstandet hiervon angeordnet sein. Die Verarbeitungseinrichtung kann auch als ein Cloud-System ausgebildet sein, das durch eine Vielzahl von, insbesondere an verschiedenen Orten angeordneten, Servern, implementiert sein kann. Die Verarbeitungseinrichtung umfasst vorzugsweise einen Speicher zur Speicherung von im Rahmen des erfindungsgemäßen Verfahrens anfallenden Daten. Die Speichereinrichtung oder eine weitere Speichereinrichtung kann zudem ein Programm speichern, das das erfindungsgemäße Verfahren implementiert. Die Verarbeitungseinrichtung umfasst zudem vorzugsweise einen Prozessor, der die Schritte des erfindungsgemäßen Verfahrens durchführen kann.

Daneben betrifft die Erfindung ein Computerprogramm, welches direkt in einem Speicher einer Verarbeitungseinrichtung ladbar ist, mit Programm-Mitteln, um die Schritte des erfindungsgemäßen Verfahrens durchzuführen, wenn das Programm in der Verarbeitungseinrichtung ausgeführt wird.

Die Erfindung betrifft auch einen elektronisch lesbaren Datenträger mit darauf gespeicherten elektronisch lesbaren Steuerinformationen, welche zumindest ein erfindungsgemäßes Computerprogramm umfassen und derart ausgestaltet sind, dass sie bei Verwendung des Datenträgers in einer Verarbeitungseinrichtung das erfindungsgemäße Verfahren durchführen.

Weitere Vorteile und Einzelheiten der Erfindung ergeben sich aus den folgenden Ausführungsbeispielen sowie den zugehörigen Zeichnungen. Hierbei zeigen schematisch:
- Figuren 1 und 2: den Ablauf eines Ausführungsbeispiels des erfindungsgemäßen Verfahrens,
- Figuren 3 und 4: das Training eines in dem in Fig.1 und 2 gezeigten Verfahren nutzbaren Modifikationsalgorithmus,
- Fig. 5: ein weiteres Ausführungsbeispiel des erfindungsgemäßen Verfahrens,
- Fig. 6: die Anzeige veränderter Bilddaten in einem Ausführungsbeispiel des erfindungsgemäßen Verfahrens, und
- Fig. 7: ein Ausführungsbeispiel eines Röntgensystems, das eine erfindungsgemäße Verarbeitungseinrichtung umfasst.

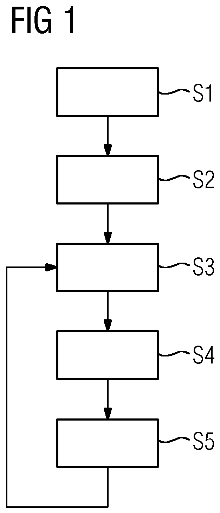

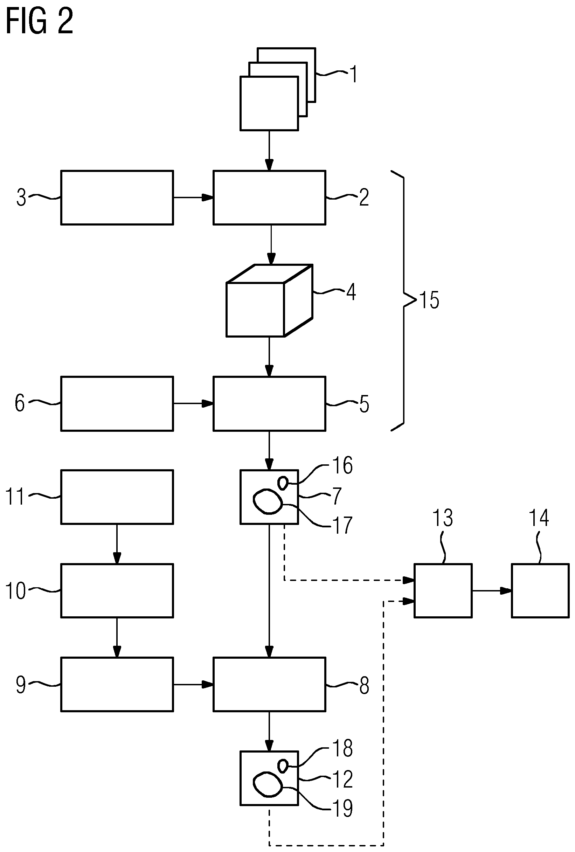

Fig. 1 zeigt ein Ablaufdiagramm eines Verfahrens zur Anpassung eines Bildeindrucks eines Bildes, wobei insbesondere ein Bildeindruck eines Bildes angepasst werden kann, das im Rahmen einer medizinischen Bildgebung ermittelt wird. Die im Rahmen des Verfahrens genutzten Daten und Verarbeitungsmodule sind schematisch in Fig. 2 dargestellt.

In Schritt S1 werden zunächst Ursprungsdaten 1 ermittelt, die beispielsweise einzelne Röntgenbilder sein können, die im Rahmen einer Computertomographie von einem Patienten aufgenommen werden. Diese werden in Schritt S2 durch einen Verarbeitungsalgorithmus 15, der von Verarbeitungsparametern 3, 6 abhängt, verarbeitet, um das Bild 7 bereitzustellen. Hierbei hängt ein Bildeindruck des Bildes 7 beziehungsweise einzelner Bildsegmente 16, 17 des Bildes 7 typischerweise von den Verarbeitungsparametern 3, 6 ab. Ein solcher Bildeindruck kann quantifiziert werden, indem ein vorgegebener Klassifikationsalgorithmus 13 auf einzelne Bildsegmente 16, 17 des Bildes 7 angewandt wird, um eine jeweilige Bildeindrucksklasse 14 zu ermitteln. Wie später noch genauer erläutert werden wird, kann ein solcher Klassifikationsalgorithmus beispielsweise durch ein Verfahren des Maschinenlernens definiert beziehungsweise parametrisiert werden.

Im gezeigten Ausführungsbeispiel umfasst der Verarbeitungsalgorithmus 15 einen Rekonstruktionsalgorithmus 2, der dreidimensionale Volumendaten 4 aus den zweidimensionalen Ursprungsdaten 1 rekonstruiert. Dieser wird durch die Verarbeitungsparameter 3 parametrisiert, die beispielsweise angeben können, welcher Rekonstruktionskernel im Rahmen der Rekonstruktion verwendet werden soll. Beispielsweise kann eine Rekonstruktion derart erfolgen, dass die Volumendaten 4 für einzelne Punkte im Volumen Hounsfield-Einheiten angeben. Es ist jedoch auch möglich, dass in den Volumendaten Kanten hervorgehoben werden. Zudem kann in Abhängigkeit der konkret genutzten Rekonstruktion beispielsweise wahlweise eine höhere Ortsauflösung oder ein besseres Signal-zu-Rausch-Verhältnis erreicht werden. Die Verarbeitungsparameter 3, von denen der Rekonstruktionsalgorithmus 2 abhängt, können somit unmittelbar den Bildeindruck und somit die Bildeindrucksklasse der einzelnen Bildsegmente 16, 17 beeinflussen.

Im Rahmen des Verarbeitungsalgorithmus 15 werden die Volumendaten 4 anschließend durch einen Abbildungsalgorithmus 5, der durch weitere Verarbeitungsparameter 6 parametrisiert wird, abgebildet, um das Bild 7 zu generieren. Der Abbildungsalgorithmus kann beispielsweise Schichtbilder oder synthetische Röntgenbilder aus den Volumendaten 4 erzeugen. Die Verarbeitungsparameter 6 können beispielsweise beschreiben, welche Schichtdicke senkrecht zu einer Bildebene bei dem Generieren von Schichtbildern berücksichtigt wird beziehungsweise inwieweit im Rahmen der Abbildung ein Weichzeichnen oder Schärfen erfolgt. Auch diese Parameter beeinflussen den Bildeindruck und somit die Bildeindrucksklasse der Bildsegmente 16, 17.

Zu verschiedenen Diagnosezwecken können hierbei unterschiedliche Bildeindrücke vorteilhaft sein. Zudem bevorzugen verschiedene Nutzer verschiedene Bildeindrücke. Um zu vermeiden, dass zur Änderung eines Bildeindrucks jeweils der komplette Verarbeitungsalgorithmus 15 erneut durchgeführt werden muss, was in einigen Fällen, beispielsweise wenn als Rekonstruktionsalgorithmus 2 als ein iterativer Rekonstruktionsalgorithmus genutzt wird, sehr rechenintensiv sein kann, kann in Schritt S3 ein Modifikationsalgorithmus 8 genutzt werden, um veränderte Bilddaten 12 zu generieren. Hierbei erfolgt die Modifikation derart, dass die Bildeindrucksklasse 14, die über den Klassifikationsalgorithmus 13 den modifizierten Bilddaten 18, 19 der jeweiligen Bildsegmente 16, 17 zugeordnet ist, einer vorgegebene Sollbildeindrucksklasse 9 entspricht.

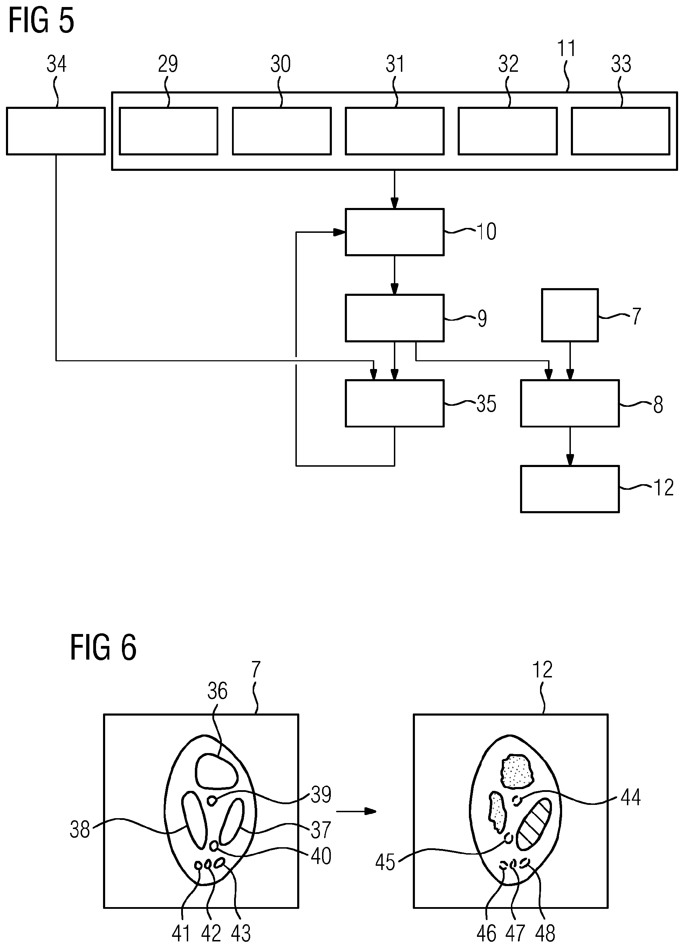

Die Sollbildeindrucksklasse 9 wird hierbei durch einen Vorgabealgorithmus 10, der, wie später noch erläutert werden wird, insbesondere durch ein Maschinenlernen parametrisiert sein kann, aus Vorgabedaten 11 ermittelt. Die Vorgabedaten 11 können den Benutzer betreffen, um den Bildeindruck an Benutzervorlieben anzugleichen. Zudem können sie die konkrete Nutzungssituation, also beispielsweise eine Darstellungs- beziehungsweise Diagnoseaufgabe, betreffen. Verschiedene mögliche Vorgabedaten werden später noch mit Bezug auf Fig. 5 diskutiert werden.

Die Modifikation des Bildes 7 kann bereits vor der Ausgabe des Bildes 7 an einen Benutzer in Schritt S4 erfolgen. Beispielsweise kann anhand von verfügbaren Vorgabedaten beziehungsweise einer Benutzerkennung bereits erkannt werden, dass ein bestimmter Bildeindruck voraussichtlich vorteilhaft sein wird. Nach der Ausgabe der modifizierten Bilddaten 12 in Schritt S4 kann in einem Schritt S5 eine Bedieneingabe erfasst werden, in Abhängigkeit der in Schritt S3 eine erneute Modifikation des Bildes 7, die gemäß der Bedieneingabe angepasst ist, durchgeführt wird. Das beschriebene Vorgehen kann so häufig wie erforderlich wiederholt werden, um einem Benutzer optimale Informationen bereitzustellen. Die in Schritt S5 erfasste Bedieneingabe kann bei der nächsten Anwendung des Modifikationsalgorithmus insbesondere als Teil der Vorgabedaten 11 berücksichtigt werden.

Das beschriebene Vorgehen kann derart modifiziert werden, dass nicht unmittelbar ein Bild 7 modifiziert wird, sondern Eingangsdaten, aus denen das Bild 7 ermittelt wird, beziehungsweise die Modifikation des Bildes kann derart erfolgen, dass eine Ermittlung des Bildes aus Eingangsdaten modifiziert wird. Die Eingangsdaten können insbesondere mehrere zweidimensionale Bilder sein, die überlagert beziehungsweise subtrahiert werden, um das Bild zu ermitteln. Beispielsweise kann es sich um ein Maskenbild und ein Kontrastmittelbild handeln, die im Rahmen einer digitalen Subtraktionsangiographie voneinander subtrahiert werden. Hierbei können die einzelnen Bilder der Eingangsdaten wie zu Fig. 2 erläutert dadurch generiert werden, dass zunächst die jeweiligen zweidimensionalen Ursprungsdaten 1 zu jeweiligen Volumendaten 4 rekonstruiert werden, aus denen jeweilig Eingangsbilder generiert werden. Dies ist beispielsweise zweckmäßig, um ein Masken- und ein Kontrastmittelbild bereitzustellen. Die Eingangsbilder können jedoch auch verschiedene aus den gleichen Volumendaten 4 generierte Bilder sein, beispielsweise unterschiedliche Schichtbilder, die überlagert dargestellt werden können. Durch eine Nutzung mehrerer zweidimensionaler Eingangsbilder zur Ermittlung des Bildes und die Modifikation dieser Bilder beziehungsweise ihrer Überlagerung kann ein resultierender Bildeindruck weiter angepasst werden, beispielsweise indem vorgegeben wird, in welchem der Segmente 16, 17 des Bildes Bilddaten welches Eingangsbildes gezeigt werden beziehungsweise wie die Bilddaten der Eingangsbilder bei einer Überlagerung gewichtet werden.

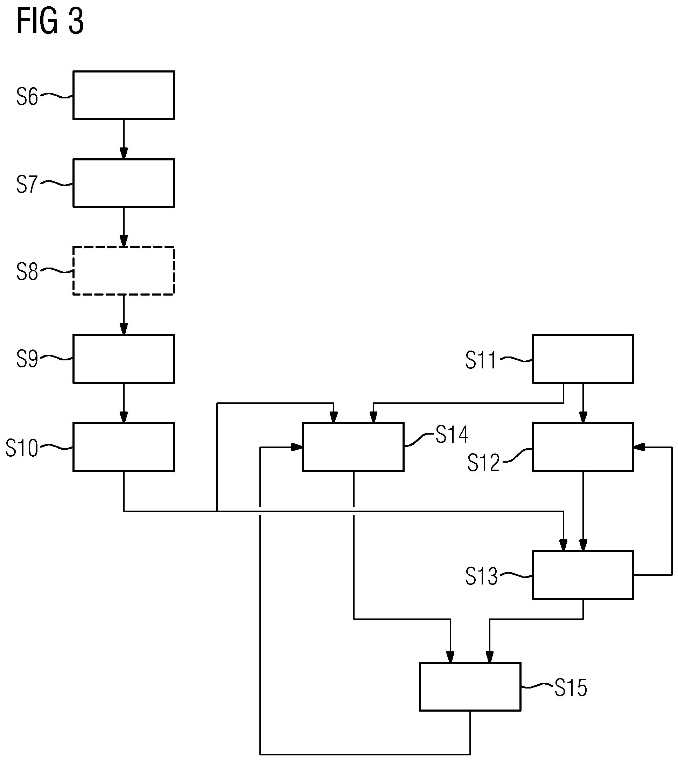

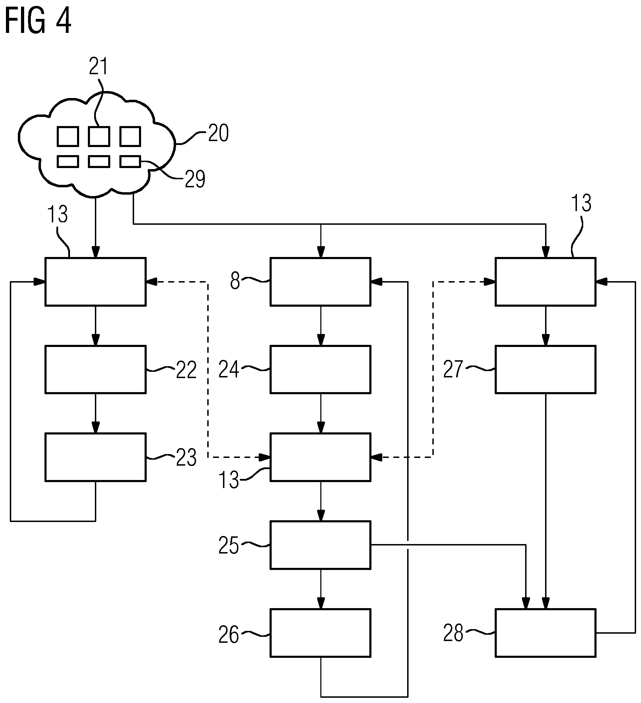

Die Figuren 3 und 4 zeigen ein Verfahren zur Bestimmung von Klassifikationsparametern des Klassifikationsalgorithmus 13 und von Modifikationsparametern des Modifikationsalgorithmus 8, das als Teil des vorangehend beschriebenen Verfahrens jedoch auch zur Vorbereitung des beschriebenen Verfahrens unabhängig von diesem durchgeführt werden kann. Hierbei zeigt Fig. 3 ein Ablaufdiagramm des Verfahrens und Fig. 4 die hierbei genutzten Datenstrukturen und Algorithmen. Die Schritte S6 bis S9 dienen hierbei zur Bereitstellung eines Trainingsdatensatzes 20, der zum Training des Klassifikationsalgorithmus 13 verwendet wird und der auch Bilder zum Training des Modifikationsalgorithmus 18 bereitstellen kann. Hierzu werden zunächst in Schritt S6 Referenzdaten erfasst. Beispielsweise können mehrere Computertomographien für verschiedene Patienten erfasst werden. Für die einzelnen Referenzdatensätze wird in Schritt S7 der zu Fig. 2 diskutierte Verarbeitungsalgorithmus 15 genutzt, um zunächst Volumendatensätze zu generieren und aus diesen zweidimensionale Abbildungen zu erzeugen. Hierbei werden die Verarbeitungsparameter 3, 6 variiert, um für jeden der in Schritt S6 erfassten Referenzdatensätze mehrere Eingangsbilder 21 zu generieren, die sich aufgrund der verschiedenen im Rahmen der Verarbeitung genutzten Verarbeitungsparameter 3, 6 bezüglich des Bildeindrucks des Gesamtbildes beziehungsweise bezüglich des Bildeindrucks einzelner Bildsegmente voneinander unterschieden.

In Schritt S8 erfolgt eine Segmentierung der Eingangsbilder 21. Soll der Klassifikationsalgorithmus ausschließlich zur Klassifikation des Bildeindrucks des Gesamtbildes trainiert werden beziehungsweise soll der Modifikationsalgorithmus ausschließlich zur Modifikation des Bildeindrucks des Gesamtbildes trainiert werden, so kann auf den Schritt S8 verzichtet werden. Die Segmentierung kann manuell durch einen oder mehrere Experten erfolgen. Besonders bevorzugt erfolgt eine Segmentierung in Schritt S8 jedoch durch einen Algorithmus zur automatischen Segmentierung der Eingangsbilder 21. Ein solcher Algorithmus kann vorangehend beispielsweise durch ein Maschinenlernen trainiert werden, indem beispielsweise für eine ausreichend große Anzahl von Bildern eine manuelle Vorsegmentierung erfolgt und der Algorithmus im Rahmen eines überwachten Lernens derart trainiert wird, dass die von ihm durchgeführte Segmentierung der vorgegebenen Segmentierung möglichst nahekommt.

In Schritt S9 wird jedem Ergebnisbild beziehungsweise, wenn vorangehend eine Segmentierung der Ergebnisbilder erfolgte, den einzelnen Segmenten des Ergebnisbildes eine jeweilige Bildeindrucksklasse zugeordnet. Es ist auch möglich, dass diese Zuordnung nicht für einzelne Segmente erfolgt, sondern für Segmenttypen, also beispielsweise für alle Segmente, die Knochen oder Teil eines Gefäßsystems zeigen. Die Zuordnung kann ausschließlich anhand der Eingangsbilder selbst manuell durch einen oder mehrere Experten erfolgen. Da jedoch typischerweise davon ausgegangen werden kann, dass zumindest für alle Segmente eines bestimmten Segmenttyps eine starke Korrelation zwischen dem Bildeindruck in den Eingangsbildern beziehungsweise Bildsegmenten und den zur Ermittlung der Bilder genutzten Verarbeitungsparametern 3, 6 existiert, ist es auch möglich, den verschiedenen Kombinationen von Verarbeitungsparametern beziehungsweise bestimmten Wertbereichen von Verarbeitungsparametern allgemein oder für einen bestimmten Segmenttyp eine bestimmte Bildeindrucksklasse 29 zuzuordnen. Hiermit kann vermieden werden, dass alle Eingangsbilder 21 manuell klassifiziert werden müssen, da es ausreicht, eine entsprechende Zuordnung zwischen Verarbeitungsparametern 3, 6 und der Bildeindrucksklasse 29 zu definieren.

Als Ergebnis des Schritts S9 liegt somit ein Trainingsdatensatz 20 vor, der eine Vielzahl von Eingangsbildern 21 und zu den jeweiligen Eingangsbildern 21 beziehungsweise zu den einzelnen Bildsegmenten der Eingangsbilder 21 zugeordneten Bildeindrucksklassen 29 umfasst. Mit Hilfe dieses Trainingsdatensatzes 20 erfolgt in Schritt S10 zunächst ein vorläufiges Training des Klassifikationsalgorithmus 13. Der Klassifikationsalgorithmus 13 wird an mehreren Stellen im beschriebenen Verfahren verwendet. Um eine möglichst übersichtliche Darstellung zu erreichen, wurde der Klassifikationsalgorithmus 13 daher in Fig. 4 mehrfach dargestellt. Es handelt sich jedoch, wie durch die gestrichelten Doppelpfeile und das identische Bezugszeichen angedeutet wird, hierbei jeweils um den gleichen Klassifikationsalgorithmus 13.

Der Klassifikationsalgorithmus 13 kann durch ein überwachtes Lernen trainiert werden. Das Training dient dazu, mehrere Klassifikationsparameter zu bestimmen. Handelt es sich bei dem Klassifikationsalgorithmus 13 beispielsweise um ein künstliches neuronales Netz, insbesondere um ein Convolutional Neural Network, so können die Klassifikationsparameter die Eingangsgewichte einzelner künstlicher Neuronen sein. Die Anfangswerte der Klassifikationsparameter können beliebig vorgegeben werden, also beispielsweise zufällig gewählt werden. Im Rahmen des Trainings wird hierbei der entsprechend parametrisierte Klassifikationsalgorithmus 13 jeweils auf wenigstens eines der Eingangsbilder 21 angewandt, um eine Bildeindrucksklasse 22 zu ermitteln. Hierbei kann die Bildeindrucksklasse 22 für das Gesamtbild und/oder für die vorangehend in Schritt S8 ermittelten Segmente ermittelt werden. Durch eine Fehlerrückführung 23 wird die ermittelte Bildeindrucksklasse 22 mit der in Schritt S9 vorgegebenen Bildeindrucksklasse 29 verglichen und in Abhängigkeit dieses Vergleichs werden die Klassifikationsparameter des Klassifikationsalgorithmus 13 angepasst. Entsprechende Verfahren zum überwachten Lernen von Klassifikationsalgorithmen sind prinzipiell bekannt und sollen daher nicht ausführlich diskutiert werden.

Nach einem ausreichenden Training des Klassifikationsalgorithmus 13, beispielsweise nach einer fest vorgegebenen Anzahl von Trainingsiterationen oder nach Erfüllung eines Konvergenzkriteriums für die Klassifikationsparameter, ist das überwachte Lernen des Klassifikationsalgorithmus 13 zunächst abgeschlossen. Die folgenden Schritte dienen dazu, ein gemeinsames unüberwachtes Lernen des Klassifikationsalgorithmus 13 und des Modifikationsalgorithmus 8 durchzuführen. Hierbei können zwar die Eingangsbilder 21 des Trainingsdatensatzes 20 weiter verwendet werden, es können jedoch beliebig auch andere Eingangsbilder genutzt werden, da die zugeordneten vorgegebenen Bildereindrucksklassen 29 nun nicht weiter benötigt werden. Die im Folgenden genutzte Lernstruktur weist hierbei die Struktur eines Generative Adversarial Network auf, wobei der Modifikationsalgorithmus 8 als Generator dient und der Klassifikationsalgorithmus 13 als Diskriminator.

In Schritt S11 werden zunächst Eingangsbilder zur Verfügung gestellt, die beispielsweise die Eingangsbilder 21 des Trainingsdatensatzes 20 oder auch andere Eingangsbilder sein können. Zudem wird für die Eingangsbilder beziehungsweise bestimmte Segmente oder Segmenttypen jeweils eine Sollbildeindrucksklasse vorgegeben, um den Modifikationsalgorithmus 8 darauf zu trainieren, diese Sollbildeindrucksklasse für das Bildsegment oder alle Segmente eines Segmenttyps zu erreichen.

In Schritt S12 wird das Eingangsbild durch den Modifikationsalgorithmus 8 verändert. Diese Veränderung hängt von den Modifikationsparametern des Modifikationsalgorithmus ab. Der Modifikationsalgorithmus kann beispielsweise ein neuronales Netz, insbesondere ein "deconvolutional" beziehungsweise entfaltendes neuronales Netz sein. In diesem Fall können die Modifikationsparameter beispielsweise die Eingangsgewichte verschiedener künstlicher Neuronen sein. Die Modifikationsparameter können zunächst beliebig, also beispielsweise zufällig, gewählt sein. Als Verarbeitungsergebnis wird ein Ergebnisbild 24 ausgegeben.

Im Schritt S13 wird der Klassifikationsalgorithmus genutzt, um die Bildeindrucksklassen des Ergebnisbildes 24 beziehungsweise der einzelnen Segmente des Ergebnisbildes 24 aufzufinden. Der Klassifikationsalgorithmus 13 ist hierbei zunächst durch die im vorangehend beschriebenen Training ermittelten Klassifikationsparameter parametrisiert. Diese können sich, wie später noch erläutert wird, jedoch im Verlauf des weiteren Trainings verändern. Soll eine Klassifikation für einzelne Bildsegmente ermittelt werden, kann das Ergebnisbild 24 vorangehend segmentiert werden. Besonders vorteilhaft ist es jedoch, wenn die in Schritt S11 bereitgestellten Eingangsbilder segmentiert werden und eine entsprechende Segmentierung auf die Ergebnisbilder 24 unverändert übertagen wird. Verschiedene Möglichkeiten zur Segmentierung wurden bereits mit Bezug auf Schritt S8 erläutert.

Die für die veränderten Bilddaten 24 beziehungsweise deren Bildsegmente ermittelten Bildeindrucksklassen 25 werden durch die Fehlerrückführung 26 mit den in Schritt S11 vorgegebenen Sollbildeindrucksklassen für das entsprechende Bildsegment verglichen und in Abhängigkeit des Vergleichsergebnisses werden die Modifikationsparameter des Modifikationsalgorithmus 8 angepasst.

Um ein robusteres Training des Modifikationsalgorithmus 8 zu erreichen, ist es vorteilhaft, gleichzeitig den Klassifikationsalgorithmus 13 derart weiter zu trainieren, dass er versucht, trotz der Modifikation durch den Modifikationsalgorithmus 8 weiterhin die gleiche Bildeindrucksklasse zu erkennen. Hierdurch kann insbesondere vermieden werden, dass der Modifikationsalgorithmus dazu trainiert wird, das Bild auf eine Weise zu verändern, die den tatsächlichen Bildeindruck kaum ändert, jedoch zu einer Fehlklassifikation des Bildeindrucks führt. Um dieses parallele Training zu erreichen, wird in Schritt S14 der Klassifikationsalgorithmus 13 auf die gleichen Eingangsbilder angewandt, die auch dem Modifikationsalgorithmus 8 zugeführt werden, um für die unveränderten Eingangsbilder beziehungsweise deren Bildsegmente eine jeweilige Bildeindrucksklasse 27 zu ermitteln. Die einzelnen Bildeindrucksklassen 27 für die unmodifizierten Eingangsbilder werden in Schritt S15 durch die weitere Fehlerrückführung 28 mit den entsprechenden Bildeindrucksklassen 25 für die Ergebnisbilder 24 verglichen. In Abhängigkeit hiervon werden die Klassifikationsparameter des Klassifikationsalgorithmus 13 angepasst, wobei die Optimierung derart erfolgen soll, dass nach Möglichkeit die Bildeindrucksklassen 27 für die Eingangsbilder beziehungsweise deren Segmente den Bildeindrucksklassen 25 für die Ergebnisbilder 24 beziehungsweise deren Segmente weitgehend entsprechen.

Die Schritte S12 bis S15 werden, beispielsweise für eine bestimmte Iterationszahl oder bis ein Konvergenzkriterium für die Modifikationsparameter und/oder die Klassifikationsparameter erfüllt wird, wiederholt. Da es sich um einen unüberwachten Lernprozess handelt, ist die Iterationszahl nicht durch die Anzahl von zur Verfügung stehenden Trainingsdatensätzen begrenzt. Es kann somit ein qualitativ hochwertiges Training des Modifikationsalgorithmus erreicht werden, wobei die Trainingsqualität allenfalls durch die Qualität eines vorangehenden Trainings des Klassifikationsalgorithmus 13 begrenzt ist.

Wie bereits zu Fig. 2 erläutert wurde, kann die Vorgabe der Solleindrucksklassen 9 für die Segmente 16, 17 des Bildes 7 durch einen Vorgabealgorithmus 10 erfolgen, der durch ein Verfahren des Maschinenlernens trainiert wird. Dies wird im Folgenden mit Bezug auf Fig. 5 an einem Ausführungsbeispiel genauer erläutert. Ziel des Trainings ist es hierbei, dass Vorgabedaten 11 durch den trainierten Verarbeitungsalgorithmus 10 automatisch ausgewertet werden, um für einen Benutzer und eine momentane Benutzungssituation besonders gut geeignete Sollbildeindrucksklassen 9 für die Bildsegmente 16, 17 des Bildes 7 zu ermitteln, so dass durch den Modifikationsalgorithmus 8 entsprechend veränderte Bilddaten 12 bereitgestellt werden können. Dieses Vorgehen wurde vorangehend bereits mit Bezug auf Fig. 1 und Fig. 2 erläutert.

Als Vorgabedaten 11 werden im gezeigten Ausführungsbeispiel eine Benutzerinformation 29, die einen Nutzer identifiziert, in dem Bild erkannte Merkmale 30, Zusatzinformationen 31 bezüglich der Bildgebung, Zusatzinformationen 32 bezüglich des Patienten und eine benutzerseitig vorgegebene Darstellungsaufgabe 33, die beispielsweise angeben soll, für welche Art von Diagnose Bilddaten bereitgestellt werden sollen, verwendet.

Der Vorgabealgorithmus 10 kann beispielsweise ein neuronales Netz sein, wobei im Rahmen des Trainings beispielsweise Parameter ermittelt werden, die Eingangsgewichte der künstlichen Neuronen des Netzes beschreiben. Vorzugsweise wird ein überwachtes Lernen genutzt, das heißt es werden zusätzlich zu den Vorgabedaten 11 zumindest in einer Trainingsphase Benutzereingaben 34 erfasst, die unmittelbar vorgeben, für welche Bildsegmente 16, 17 beziehungsweise Segmenttypen welche Sollbildeindrucksklassen 9 in den veränderten Bilddaten 12 vorliegen soll. Die durch den Vorgabealgorithmus 10 ermittelten Sollbildeindrucksklassen können dann im Rahmen einer Fehlerrückführung 35 mit den durch die Benutzereingabe 34 vorgegebenen Sollbildeindrucksklassen verglichen werden. Die Parametrisierung des Vorgabealgorithmus 10 kann entsprechend angepasst werden, um Abweichungen zwischen diesen Informationen zu minimieren.

Nach einer anfänglichen Lernphase kann auf die zusätzliche Eingabe 34 verzichtet werden. Es soll jedoch möglich sein, dass der Benutzer die Eingabe 34 weiterhin tätigen kann, beispielsweise dann, wenn die automatisch vorgegebenen Sollbildeindrucksklassen 9 seines Erachtens nicht geeignet sind, den Bildeindruck bedarfsgerecht anzupassen. Hierbei kann dann, wenn eine Benutzereingabe 34 erfolgt, diese genutzt werden, um den Vorgabealgorithmus 10 weiter zu trainieren.

Fig. 6 zeigt schematisch ein Beispiel für eine Veränderung eines Bildes 7 durch den Modifikationsalgorithmus 8. Das ursprüngliche Bild 7 zeigt hierbei eine Vielzahl von Merkmalen 36 bis 43, die ungefähr mit gleicher Deutlichkeit zu erkennen sind. Möchte ein Benutzer nun beispielsweise für einen bestimmten Diagnosezweck oder für eine Visualisierung für einen Patienten das Merkmal 37 hervorheben, können Sollbildeindrucksklassen für die Segmente, die die einzelnen Merkmale 36 bis 43 umfassen, vorgegeben werden, so dass das Merkmal 37 hervorgehoben wird, also beispielsweise mit besonders starkem Kontrast und besonders scharf, dargestellt wird. Die Merkmale 36, 38 können hierbei beispielsweise weichgezeichnet werden, so dass sie einem Betrachter zwar weiterhin Informationen über die Lage des Merkmals 37 geben können, jedoch nicht von diesem Merkmal 37 ablenken. Die relativ kleinen Merkmale 39 bis 43 können in den veränderten Bilddaten 12 nicht oder kaum erkennbar sein. Um einen Benutzer dennoch darauf hinzuweisen, dass diese Merkmale vorhanden sind und durch Wahl einer anderen Bildmodifikation sichtbar gemacht beziehungsweise hervorgehoben werden können, können die Bildbereiche 44 bis 48, in denen sich die in den veränderten Bilddaten 12 nicht oder kaum erkennbaren Merkmale 39 bis 43 befinden, markiert werden, beispielsweise indem die entsprechenden Bereiche mit einer farbigen gestrichelten Linie umgeben werden.

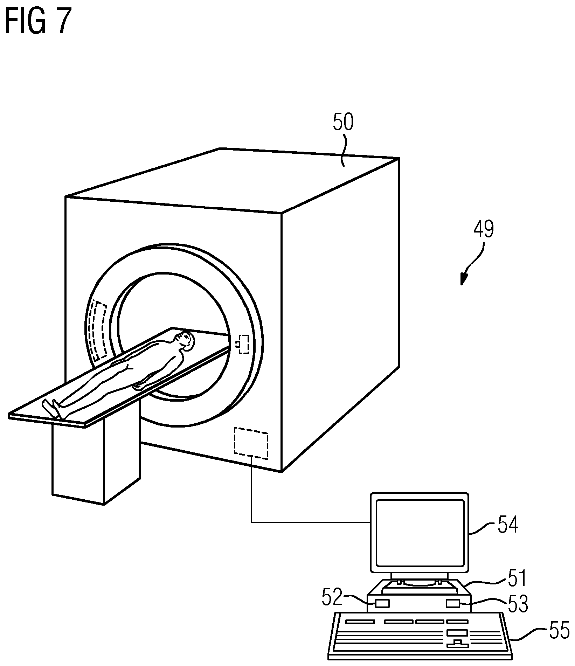

Fig. 7 zeigt ein Röntgensystem 49, das eine Röntgeneinrichtung 50, nämlich einen Computertomographen, und eine Verarbeitungseinrichtung 51 umfasst. Die Verarbeitungseinrichtung ist dazu eingerichtet, die von der Röntgeneinrichtung 50 bereitgestellten Ursprungsdaten zu verarbeiten, um Bilder, insbesondere Schnittbilder, an einer Anzeigeeinrichtung 54 für einen Benutzer darzustellen. Bedarfsgerecht kann die Verarbeitungseinrichtung 51 hierbei gemäß dem vorangehend erläuterten Verfahren die Bilder durch einen Modifikationsalgorithmus modifizieren, um Sollbildeindrucksklassen für einzelne Bildsegmente oder Segmenttypen vorzugeben. Hierbei kann bereits bei einem ersten anzeigen des Bildes eine Modifikation erfolgen. Um dies zu erreichen, kann beispielsweise ein ähnlicher Ansatz genutzt werden, wie er mit Bezug zu Fig. 5 diskutiert wurde, wobei ein Training des Vorgabealgorithmus 10 derart erfolgt, dass die Vorgabe einer Darstellungsaufgabe 33 nicht erforderlich ist. Ist ein Benutzer mit dem resultierenden modifizierten Bild nicht zufrieden beziehungsweise möchte er beispielsweise für einen anderen Diagnosezweck eine andere Bildmodifikation angezeigt bekommen, kann er durch ein Bedienmittel 55 eine entsprechende Darstellungsaufgabe oder eine gewünschte Solleindrucksklasse für das gesamte Bild oder für Segmente des Bilds vorgeben.

Das erläuterte Verfahren kann durch ein Computerprogramm implementiert sein, das in einen Speicher 52 der Verarbeitungseinrichtung 51 ladbar ist. Die Verfahrensschritte können durch einen Prozessor 53 ausgeführt werden. Das Computerprogramm kann auf einem elektronisch lesbaren Datenträger, beispielsweise einer CD-ROM, DVD, Festplatte, einem USB-Stick oder Ähnlichem bereitgestellt werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Computer-implementiertes Verfahren zur Anpassung eines Bildeindrucks eines Bildes (7), insbesondere eines im Rahmen einer medizinischen Bildgebung ermittelten Bildes (7), umfassend die Schritte:
- Bereitstellen eines Bildes (7) oder von Eingangsdaten, aus denen das Bild (7) ermittelt wird,
- Vorgabe einer jeweiligen Sollbildeindrucksklasse (9) für wenigstens ein direkt oder durch Vorgabe wenigstens eines Segmenttyps vorgegebenes Bildsegment (16, 17) des Bildes (7), wobei ein Zusammenhang zwischen den Bilddaten des jeweiligen Bildsegments (16, 17) und einer zugeordneten Bildeindrucksklasse (14) durch einen Klassifikationsalgorithmus (13) vorgegeben ist,
- Verändern des Bildes (7) oder der Eingangsdaten durch einen Modifikationsalgorithmus (8), um die den hieraus resultierenden, veränderten Bilddaten (12, 18, 19) des jeweiligen Bildsegments (16, 17) zugeordnete Bildeindrucksklasse (14) der jeweiligen Sollbildeindrucksklasse (9) anzugleichen, wobei wenigstens ein Modifikationsparameter des Modifikationsalgorithmus (8) in Abhängigkeit des Klassifikationsalgorithmus (13) vorgegeben ist oder wird,
- wobei für verschiedene Bildsegmente (16, 17) und/oder verschiedene Segmenttypen verschiedene Sollbildeindrucksklassen (9) vorgegeben werden,
**dadurch gekennzeichnet, dass**
- das Bild (7) oder die Eingangsdaten durch einen Verarbeitungsalgorithmus (15), der von wenigstens einem Verarbeitungsparameter (3, 6) abhängt, aus Ursprungsdaten (1) ermittelt sind oder werden, wobei die Bildeindrucksklasse (14) des jeweiligen Bildsegments (16, 17) und/oder Segmenttyps von dem Verarbeitungsparameter (3, 6) abhängt,
- und der Verarbeitungsalgorithmus (15) einen Rekonstruktionsalgorithmus (2) zur Rekonstruktion von dreidimensionalen Volumendaten (4) aus zweidimensionalen Ursprungsdaten (1), insbesondere aus Röntgenbildern, umfasst, wobei der Rekonstruktionsalgorithmus (2) von dem Verarbeitungsparameter (3) oder von wenigstens einem der Verarbeitungsparameter (3, 6) abhängt und/oder wobei das Bild (7) oder die Eingangsdaten durch einen Abbildungsalgorithmus (5) aus den Volumendaten (4) generierte zweidimensionale Bilddaten sind, wobei der Abbildungsalgorithmus (5) von dem Verarbeitungsparameter (6) oder wenigstens einem der Verarbeitungsparameter (3, 6) abhängt.

2. Verfahren nach Anspruch 1, **dadurch gekennzeichnet, dass** der Modifikationsparameter durch ein Verfahren des Maschinenlernens ermittelt ist oder wird.

3. Verfahren nach einem der vorangehenden Ansprüche, **dadurch gekennzeichnet, dass** wenigstens ein Klassifikationsparameter des Klassifikationsalgorithmus (13) und der Modifikationsparameter gemeinsam durch ein Verfahren des Maschinenlernens ermittelt sind oder werden.

4. Verfahren nach Anspruch 3, **dadurch gekennzeichnet, dass** im Rahmen des Maschinenlernens ein Lernalgorithmus zugleich oder abwechselnd versucht, den Modifikationsparameter derart zu wählen, dass bei einem Anwenden des Klassifikationsalgorithmus (13) auf ein direkt oder durch Vorgabe wenigstens eines Segmenttyps vorgegebenes Bildsegment eines Ergebnisbildes, das durch Anwenden des Modifikationsalgorithmus (13) auf ein Eingangsbild (21) ermittelt wird, eine vorgegebene Sollbildeindrucksklasse ermittelt wird, und den Klassifikationsparameter derart zu wählen, dass bei einem Anwenden des Klassifikationsalgorithmus (13) auf das wenigstens eine Bildsegment des Ergebnisbildes und auf ein dem Bildsegment des Ergebnisbildes zugeordnetes Bildsegment des Eingangsbildes (21) die gleiche Bildeindrucksklasse (25, 27) ermittelt wird.

5. Verfahren nach einem der vorangehenden Ansprüche, **dadurch gekennzeichnet, dass** die Sollbildeindrucksklasse (9) und/oder das wenigstens eine Bildsegment (16, 17) und/oder der wenigstens eine Segmenttyp, für das oder den die Sollbildeindrucksklasse (9) vorgegeben wird, in Abhängigkeit einer Bedieneingabe (33, 34) eines Benutzers und/oder einer den Benutzer identifizierenden Benutzerinformation (29) und/oder des Bildes (7) und/oder der Eingangsdaten und/oder der Ursprungsdaten (1) und/oder einer die Bildgebung des Bildes (7) und/oder einen in dem Bild (7) abgebildeten Patienten betreffenden Zusatzinformation (31, 32) vorgegeben werden.

6. Verfahren nach einem der vorangehenden Ansprüche, **dadurch gekennzeichnet, dass** die Vorgabe der Sollbildeindrucksklasse (9) und/oder des wenigstens einen Bildsegments (16, 17) und/oder Segmenttyps, für das oder den die Sollbildeindrucksklasse (9) vorgegeben wird, und/oder eine Vorgabe von durch den oder einen Benutzer wählbaren oder diesem empfohlenen Sollbildeindrücken (9) und/oder Bildsegmenten (16, 17) und/oder Segmenttypen, für die Sollbildeindrücke (9) wählbar sind oder empfohlen oder vorgegeben werden, durch einen Vorgabealgorithmus (10) erfolgt, der durch ein Maschinenlernen parametrisiert ist oder wird.

7. Verfahren nach einem der vorangehenden Ansprüche, **dadurch gekennzeichnet, dass** der Modifikationsalgorithmus (8) dazu eingerichtet ist, in Abhängigkeit einer für das Bild (7) vorgegebenen Darstellungsaufgabe (33) automatisch Bildsegmente (16, 17) und/oder Segmenttypen und jeweils zugeordnete Sollbildeindrucksklassen (9) vorzugeben.

8. Verfahren nach einem der vorangehenden Ansprüche, **dadurch gekennzeichnet, dass** das Bild (7) oder die veränderten Bilddaten (12, 18, 19) für den oder einen Benutzer dargestellt werden, wobei wenigstens ein Bildbereich (44 - 48) markiert wird, der ein Merkmal (39 - 43) umfasst, dessen Darstellung von der Bildeindrucksklassifikation (14) des Bildes (7) oder wenigstens eines Segments (16, 17) des Bildes (7) oder von der Darstellungsaufgabe (33) abhängt.

9. Verfahren nach einem der vorangehenden Ansprüche, **dadurch gekennzeichnet, dass** das Bild (7) durch eine überlagerte Darstellung, insbesondere durch eine Subtraktion, von durch die Eingangsdaten beschreibenden Quellbildern ermittelt wird, wobei der Modifikationsalgorithmus (8) die Quellbilder als Eingangsdaten verarbeitet.

10. Verarbeitungseinrichtung zur Verarbeitung von Bildern (7), **dadurch gekennzeichnet, dass** sie zur Durchführung des Verfahrens nach einem der vorangehenden Ansprüche eingerichtet ist.

11. Computerprogramm, welches direkt in einen Speicher (52) einer Verarbeitungseinrichtung (51) ladbar ist, mit Programm-Mitteln, um die Schritte des Verfahrens nach einem der Ansprüche 1 bis 9 durchzuführen, wenn das Programm in der Verarbeitungseinrichtung (51) ausgeführt wird.

12. Elektronisch lesbarer Datenträger mit darauf gespeicherten elektronisch lesbaren Steuerinformationen, welche zumindest ein Computerprogramm nach Anspruch 11 umfassen und derart ausgestaltet sind, dass sie bei Verwendung des Datenträgers in einer Verarbeitungseinrichtung (51) das Verfahren nach einem der Ansprüche 1 bis 9 durchführen.

## Claims

1. Computer-implemented method for adapting an image impression of an image (7), in particular of an image (7) acquired in the context of medical imaging, including the steps:
- providing an image (7) or input data from which the image (7) is acquired,
- specifying a respective ideal image impression class (9) for at least one image segment (16, 17) of the image (7) that has been specified directly or by specifying at least one segment type, wherein a connection between the image data in the respective image segment (16, 17) and an assigned image impression class (14) is specified by a classification algorithm (13),
- modification of the image (7) or of the input data by a modification algorithm (8), in order to adapt the image impression class (14) assigned to the resulting modified image data (12, 18, 19) pertaining to the respective image segment (16, 17) to match the respective ideal image impression class (9), wherein at least one modification parameter of the modification algorithm (8) is or becomes specified as a function of the classification algorithm (13),
- wherein different ideal image impression classes (9) are specified for different image segments (16, 17) and/or different segment types,
**characterised in that**
- the image (7) or the input data is or are acquired from original data (1) by a processing algorithm (15), which is dependent on at least one processing parameter (3, 6), wherein the image impression class (14) of the respective image segment (16, 17) and/or of the segment type is dependent on the processing parameter (3, 6),
- and the processing algorithm (15) includes a reconstruction algorithm (2) for reconstructing three-dimensional volume data (4) from two-dimensional original data (1), in particular from X-ray images, wherein the reconstruction algorithm (2) depends on the processing parameter (3) or on at least one of the processing parameters (3, 6) and/or wherein the image (7) or the input data are two-dimensional image data generated by a mapping algorithm (5) from the volume data (4), wherein the mapping algorithm (5) is dependent on the processing parameter (6) or on at least one of the processing parameters (3, 6).

2. Method according to claim 1, **characterised in that** the modification parameter is or becomes determined by a method of machine learning.

3. Method according to one of the preceding claims, **characterised in that** at least one classification parameter of the classification algorithm (13) and the modification parameter are or become determined together by a method of machine learning.

4. Method according to claim 3, **characterised in that**, in the context of machine learning, a learning algorithm tries at the same time or alternately to select the modification parameter such that when the classification algorithm (13) is applied to an image segment, which has been specified directly or by specifying at least one segment type, of a resulting image, which is determined by applying the modification algorithm (13) to an input image (21), a specified ideal image impression class is determined, and tries to select the classification parameter such that when the classification algorithm (13) is applied to the at least one image segment of the resulting image and to an image segment of the input image (21) that is assigned to the image segment of the resulting image, the same image impression class (25, 27) is determined.

5. Method according to of the preceding claims, **characterised in that** the ideal image impression class (9) and/or the at least one image segment (16, 17) and/or the at least one segment type, for which the ideal image impression class (9) is specified, is specified as a function of a user input (33, 34) by a user and/or of user information (29) identifying the user and/or of the image (7) and/or of the input data and/or of the original data (1) and/or of additional information (31, 32) relating to the imaging of the image (7) and/or to a patient shown in the image (7).

6. Method according to one of the preceding claims, **characterised in that** the specification of the ideal image impression class (9) and/or of the at least one image segment (16, 17) and/or segment type, for which the ideal image impression class (9) is specified, and/or a specification of ideal image impressions (9) that can be selected by the or by a user or have been recommended to them and/or image segments (16, 17) and/or segment types for which ideal image impressions (9) are selectable or recommended or specified ensues by means of a specifying algorithm (10), which is or becomes parameterised by machine learning.

7. Method according to one of the preceding claims, **characterised in that** the modification algorithm (8) is configured to automatically specify image segments (16, 17) and/or segment types and ideal image impression classes (9) that have been assigned in each case, as a function of a viewing task (33) that has been specified for the image (7).

8. Method according to one of the preceding claims, **characterised in that** the image (7) or the modified image data (12, 18, 19) are shown for the or a user, wherein at least one image region (44 - 48) is highlighted, which includes a feature (39 - 43), the representation of which depends on the image impression classification (14) of the image (7) or at least of one segment (16, 17) of the image (7) or on the viewing task (33).

9. Method according to one of the preceding claims, **characterised in that** the image (7) is acquired by means of a superimposed view, in particular by subtraction, from source images that describe the input data, wherein the modification algorithm (8) processes the source images as input data.

10. Processing device for processing images (7), **characterised in that** it is configured to carry out the method according to one of the preceding claims.

11. Computer program, which can be loaded directly into a memory (52) of a processing device (51), with programming means to carry out the steps in the method according to one of claims 1 to 9 when the program is run in the processing device (51).

12. Electronically readable data carrier with electronically readable control information stored thereon, which includes at least one computer program according to claim 11 and is embodied such that, when the data carrier is used in a processing device (51), it runs the method according to one of claims 1 to 9.

## Revendications

1. Procédé mis en œuvre par ordinateur d'ajustement de l'impression d'une image (7), en particulier d'une image (7) déterminée dans le cadre d'une imagerie médicale, comprenant les stades :
- mise à disposition d'une image (7) ou de données d'entrée, à partir desquelles on détermine l'image (7),
- assertion d'une classe (9) d'impression respective d'image de consigne pour au moins un segment (16, 17) de l'image (7) donné à l'avance directement ou par assertion d'au moins un type de segment, dans lequel une relation entre les données d'image du segment (16, 17) d'image respectif et une classe (14) d'impression d'image affectée est donnée à l'avance par un algorithme (13) de classement,
- modification de l'image (7) ou des données d'entrée par un algorithme (8) de modification, afin d'égaler, à la classe (9) respective d'impression d'image de consigne, la classe (14) d'impression d'image affectée aux données (12, 18, 19) d'image modifiées en résultant du segment (16, 17) d'image respectif, dans lequel on donne ou est donné à l'avance au moins un paramètre de modification de l'algorithme (8) de modification en fonction de l'algorithme (13) de classement,
- dans lequel, pour divers segments (16, 17) d'image et/ou divers types de segments, on donne à l'avance diverses classes (9) d'impression d'image de consigne,
**caractérisé en ce qu'**
- on détermine ou sont déterminés, à partir de données (1) d'origine, l'image (7) ou les données d'entrée par un algorithme (15) de traitement, qui dépend d'au moins un paramètre (3, 6) de traitement, dans lequel la classe (14) d'impression d'image du segment (16, 17) d'image respectif et/ou du type de segment dépend du paramètre (3, 6) de traitement,
- et l'algorithme (15) de traitement comprend un algorithme (2) de reconstruction pour la reconstruction de données (4) en volume en trois dimensions à partir de données (1) d'origine en deux dimensions, en particulier à partir d'image de rayons X, dans lequel l'algorithme (2) de reconstruction dépend du paramètre (3) de traitement ou d'au moins l'un des paramètres (3, 6) de traitement et/ou dans lequel l'image (7) ou les données d'entrée sont des données d'image en deux dimensions créées par un algorithme (5) de représentation à partir des données (4) en volume, dans lequel l'algorithme (5) de représentation dépend du paramètre (6) de traitement ou d'au moins l'un des paramètres (3, 6) de traitement.

2. Procédé suivant la revendication 1, **caractérisé en ce que** l'on détermine ou est déterminé le paramètre de modification par un procédé de l'apprentissage par machine.

3. Procédé suivant l'une des revendications précédentes, **caractérisé en ce que** l'on détermine ou sont déterminés le au moins un paramètre de classification de l'algorithme (13) de classification et le paramètre de modification conjointement par un procédé de l'apprentissage par machine.

4. Procédé suivant la revendication 3, **caractérisé en ce que**, dans le cadre de l'apprentissage par machine, un algorithme d'apprentissage cherche, en même temps ou en alternance, à choisir le paramètre de modification de manière à déterminer, lors d'une application de l'algorithme (13) de classement à un segment d'une image de résultat, qui est donné à l'avance directement ou par l'assertion d'au moins un type de segment, qui est déterminé par l'application de l'algorithme (13) de modification à une image (21) d'entrée, une classe d'impression d'image de consigne donnée à l'avance et de manière à choisir le paramètre de classement de manière, lors d'une application de l'algorithme (13) de classement au au moins un segment de l'image de résultat et à un segment, affecté au segment de l'image de résultat, de l'image (21) d'entrée, à déterminer la même classe (25, 27) d'impression d'image.

5. Procédé suivant l'une des revendications précédentes, **caractérisé en ce que** l'on donne à l'avance la classe (9) d'impression d'image de consigne et/ou le au moins un segment (16, 17) d'image et/ou le au moins un type de segment pour lequel la classe (9) d'impression d'image de consigne est donnée à l'avance, en fonction d'une entrée (33, 34) d'opération d'un utilisateur et/ou d'une information (29) d'utilisateur, identifiant l'utilisateur, et/ou de l'image (7) et/ou des données d'entrée et/ou des données (1) d'origine et/ou à une information (31, 32) supplémentaire concernant l'imagerie de l'image (7) et/ou un patient représenté dans l'image (7).

6. Procédé suivant l'une des revendications précédentes, **caractérisé en ce que** l'on donne à l'avance l'assertion de la classe (9) d'impression d'image de consigne et/ou du au moins un segment (16, 17) d'image et/ou du type de segment pour lequel la classe (9) d'impression d'image de consigne est donnée à l'avance et/ou une assertion d'impressions (9) d'image de consigne, pouvant être choisies par le ou un utilisateur ou à lui recommandées, et/ou de segments (16, 17) d'image et/ou de types de segment, pour lesquels des impressions (9) d'image de consigne peuvent être choisies ou recommandées ou données à l'avance, s'effectue par un algorithme (10) d'assertion, qui est paramétré par un algorithme d'assertion.

7. Procédé suivant l'une des revendications précédentes, **caractérisé en ce que** l'algorithme (8) de modification est agencé pour, en fonction d'une tâche (33) de représentation donnée à l'avance pour l'image (7), donner à l'avance automatiquement des segments (16, 17) d'image et/ou des types de segment et respectivement des classes (9) d'impression d'image de consigne affectées respectivement.

8. Procédé suivant l'une des revendications précédentes, **caractérisé en ce que** l'on représente l'image (7) ou les données (12, 18, 19) d'image modifiées pour le ou un utilisateur, dans lequel on repère au moins une partie (44 - 48) d'image, qui comprend une caractéristique (39 - 43), dont la représentation dépend du classement (14) d'impression d'image de l'image (7) ou d'au moins un segment (16, 17) de l'image (7) ou de la tâche (33) de représentation.

9. Procédé suivant l'une des revendications précédentes, **caractérisé en ce que** l'on détermine l'image (7) par une représentation superposée, en particulier par une soustraction, d'images sources décrites par les données d'entrée, dans lequel l'algorithme (8) de modification traite les images sources comme données d'entrée.

10. Dispositif de traitement pour le traitement d'images (7), **caractérisé en ce qu'**il est agencé pour effectuer le procédé suivant l'une des revendications précédentes.

11. Programme d'ordinateur, qui peut être chargé directement dans la mémoire (52) d'un dispositif (51) de traitement, comprenant des moyens de programme pour exécuter les stades du procédé suivant l'une des revendications 1 à 9, lorsque le programme est exécuté dans le dispositif (51) de traitement.

12. Support de données exploitables électroniquement ayant des informations de commande exploitables électroniquement, qui y sont mises en mémoire, qui comprennent au moins un programme d'ordinateur suivant la revendication 11 et qui sont conformées de manière à exécuter, lors de l'utilisation du support de données dans un dispositif (51) de traitement, le procédé suivant l'une des revendications 1 à 9.
